(19) Europäisches Patentamt / European Patent Office / Office européen des brevets

(11) **EP 2 458 384 A2**

(12) **DEMANDE DE BREVET EUROPEEN**

(43) Date de publication:
30.05.2012 Bulletin 2012/22

(51) Int Cl.:
*G01N 33/576* (2006.01)   *G01N 33/68* (2006.01)
*G06F 19/24* (2011.01)   *C12Q 1/37* (2006.01)

(21) Numéro de dépôt: 11175060.0

(22) Date de dépôt: 13.05.2005

(84) Etats contractants désignés:
AT BE BG CH CY CZ DE DK EE ES FI FR GB GR HU IE IS IT LI LT LU MC NL PL PT RO SE SI SK TR

(30) Priorité: 14.05.2004 FR 0405306
28.10.2004 FR 0411536
28.10.2004 US 622886 P

(62) Numéro(s) de document de la (des) demande(s) initiale(s) en application de l'article 76 CBE:
05771179.8 / 1 745 403

(27) Demande déposée antérieurement:
13.05.2005 PCT/FR2005/001217

(71) Demandeurs:
• UNIVERSITE D'ANGERS
49000 Angers (FR)

• Centre Hospitalier Universitaire
d'Angers
49100 Angers (FR)

(72) Inventeur: Calès, Paul
49240 Avrillé (FR)

(74) Mandataire: Icosa
83 avenue Denfert-Rochereau
75014 Paris (FR)

Remarques:
Cette demande a été déposée le 22-07-2011 comme demande divisionnaire de la demande mentionnée sous le code INID 62.

(54) **Méthode pour diagnostiquer la présence et/ou la sévérité d'une pathologie hépatique chez un sujet et/ou pour suivre l'efficacité d'un traitement d'une telle pathologie**

(57) Méthode pour diagnostiquer la présence et/ou la sévérité d'une pathologie hépatique et/ou pour suivre l'efficacité d'un traitement curatif contre une pathologie hépatique chez un sujet comprenant l'établissement d'au moins un score diagnostique non-invasif, en particulier d'un score diagnostique de la fibrose portale et septable et/ou un score estimateur de l'aire de fibrose, et/ou d'un score estimateur de la dimension fractale.

Courbe ROC SNIFF 7b°

FIGURE 1

EP 2 458 384 A2

**Description**

La présente invention concerne le domaine du diagnostic en hépatologie, et a notamment pour objet une méthode permettant l'évaluation de la présence et/ou de la sévérité la fibrose hépatique du foie, ou encore l'évaluation de l'aire de fibrose, ou encore l'évaluation de l'architecture du foie (score de fibrose et dimension fractale).

Au sens de la présente invention, le terme « évaluation de la présence de la fibrose » signifie qu'il est recherché si une fibrose existe ou non chez le patient testé par la méthode de l'invention; le terme « évaluation de la sévérité » signifie qu'il est recherché une mesure du degré de la fibrose, ceci doit être distingué de la sévérité de l'atteinte hépatique, qui est une déficience fonctionnelle du foie. Le terme « évaluation de l'aire de fibrose » signifie qu'il est recherché une mesure du degré lésionnel du foie par la fibrose. Il est précisé que la déficience fonctionnelle du foie est fonction du degré lésionnel anatomique du foie, mais pas linéairement.

La gravité des maladies chroniques du foie réside dans la fibrose qui est une cicatrice secondaire à l'inflammation. Les causes des maladies fibrosantes du foie sont principalement les infections virales B et C, l'alcool et la stéatose (foie gras).

Jusqu'à présent, l'évaluation de la fibrose reposait sur la ponction biopsie hépatique (PBH). La fibrose hépatique se classe d'après la PBH en score semi-quantitatif de fibrose. Il existe plusieurs classifications basées sur l'observation de lésions similaires. La description de ces lésions est principalement qualitative d'après un trouble (ou distorsion) de l'architecture de l'unité élémentaire (au niveau fonctionnel et anatomique) du foie qu'est le « lobule » hépatique. La fibrose naît en périphérie du lobule dans l'espace « porte » (stade F1) pour s'étendre au sein du lobule (bandes restreintes de fibrose ou stade F2) puis le disséquer (bandes extensives de fibrose ou stade F3) pour être concentrique et isoler les cellules hépatiques (stade F4 ou cirrhose). La classification Métavir décrite ci-dessus (Bedossa et al, 1994, Hepatology, vol. 20, pages 15-20) est une des plus utilisées. Elle classe la fibrose hépatique en 5 stades de F0 à F4, le stade F4 correspondant au stade ultime de cirrhose. La fibrose est dite cliniquement significative lorsqu'elle est de stade F $\geq$ 2. Le score de fibrose F est utilisé par tous les spécialistes du foie dans le monde (selon des classifications différentes). C'est le paramètre le plus important pour déterminer la gravité d'une maladie du foie, son potentiel évolutif et l'indication du traitement. Il est d'une aide déterminante pour pouvoir prescrire un traitement ou pour une prise en charge d'un malade. Cette classification en score F est semi-quantitative pour 3 rasons : a) la description des lésions est purement qualitative et donc évaluée par un médecin anatomo-pathologiste, b) la gradation ne peut être faite qu'en un nombre fini et restreint de stades (de 4 à 6 sans compter l'absence de fibrose), c) la progression de la quantité de fibrose n'est pas linéaire en fonction des stades. L'aspect quantitatif est dû au caractère ordonné des classes suivant l'extension de la fibrose au sein du lobule.

Il existe un moyen purement quantitatif de mesurer la fibrose : c'est la mesure de l'aire (ou surface) de fibrose par une technique semi-automatique appelée analyse d'image. Par rapport à un panel de marqueurs sanguins de fibrose, considéré comme référence, l'aire de fibrose s'est avérée être une mesure plus fiable que le score de Métavir (Pilette et al, 1998, J Hepatol, vol. 28, pages 439-46).

Cependant, la PBH est un examen coûteux et invasif donc susceptible de complications et nécessitant au minimum une hospitalisation de jour. Les contraintes actuelles de la PBH (coût, intervention invasive nécessitant une hospitalisation) en limitent l'utilisation. Faire appel à cette méthode diagnostique demeure l'usage pratiquement exclusif de spécialistes du foie. De ce fait, la prise en charge médicale actuelle concerne des malades qui souvent sont à un stade relativement avancé de la maladie (cirrhose souvent compliquée), pour lesquels les possibilités de traitement sont moindres.

Plusieurs enquêtes démontrent clairement que la PBH est le principal facteur limitant du dépistage et de l'accès aux traitements. La mise au point d'alternatives à la PBH, qui est le but de la présente invention, fait partie des recommandations de recherche des conférences de consensus américaine et française en 2002.

La fibrose hépatique, y compris jusqu'au stade de cirrhose récente, est un état réversible. Un dépistage précoce de la fibrose permet souvent de proposer des mesures permettant de guérir la maladie ou au moins d'en limiter les conséquences.

Les alternatives à la PBH sont les moyens non-invasifs, au premier rang desquels les marqueurs sanguins de fibrose. Le terme de marqueurs sanguins de fibrose possède en fait deux significations. Pour le biologiste, il s'agit de marqueurs reflétant l'un des processus dynamiques de la fibrose : fibrogenèse (production de la fibrose), fibrolyse (destruction de la fibrose). Pour le clinicien, il s'agit d'un marqueur du degré de fibrose observé à l'examen anatomopathologique (fibrose « septale » principalement), c'est-à-dire une image statique résultant des deux processus dynamiques précédents. De plus, le clinicien distingue ces indicateurs en marqueurs directs lorsqu'ils sont issus d'une des molécules impliquées dans la matrice extracellulaire (fibrose) et en marqueurs indirects en tant que reflets mais non partie intégrante de cette fibrose visible.

La demande de brevet internationale publiée sous le numéro WO 02/16949 décrit une méthode de diagnostic de maladies inflammatoires, fibrotiques ou cancéreuses, dans laquelle on mesure les valeurs de marqueurs biochimiques dans le sérum ou le plasma d'un patient, on combine lesdites valeurs grâce à une fonction logistique, et on analyse la valeur finale de ladite fonction logistique en vue de déterminer la présence de fibrose ou la présence de lésion nécrotico-inflammatoires du foie. Cette demande de brevet internationale permet de proposer un test de fibrose. Toutefois, les marqueurs utilisés sont des marqueurs biochimiques classiques (marqueurs indirects), qui ne sont pas des indicateurs spécifiques de la fibrose et peuvent varier en fonction d'autres perturbations présentes au cours des maladies du foie.

Le test mis sur le marché correspondant à la méthode du brevet WO02/16949, (voir aussi Imbert-Bismut et al, Lancet 2001, Vol. 37, pages 1069-1075), appelé le Fibrotest commercialisé par la société Biopredictive présente notamment l'inconvénient d'avoir des difficultés à classer correctement les malades ayant des hépatites virales de stade F0 et F4.

De plus, la demande de brevet internationale publiée sous le numéro WO03/073822 concerne une méthode pour diagnostiquer la présence ou la sévérité d'une fibrose hépatique chez un patient. Cette méthode est basée sur la détection de trois marqueurs que sont 1'α2-macroglobuline, l'acide hyaluronique et l'inhibiteur tissulaire des métalloprotéinases-1.

La présente invention a pour objet de proposer de nouveaux outils permettant de déterminer les stades de fibrose F, en particulier de score $F \geq 2$, et de quantifier de manière fine le degré exact de cette fibrose, en vue de diagnostiquer la présence et/ou la sévérité d'une pathologie hépatique, et/ou pour suivre l'efficacité d'un traitement curatif.

Le suivi de l'efficacité d'un traitement curatif ou suspensif est important. Comme la plupart des maladies chroniques du foie s'accompagnent d'une fibrose, le traitement curatif ou suspensif a pour effet de ralentir la progression voire de faire régresser la fibrose. Il est donc important de pouvoir disposer de tests pouvant évaluer cette variation de fibrose.

Contrairement aux outils et méthodes de l'art antérieur, la présente invention ne concerne pas seulement les fibroses dont la cause est virale, mais aussi les fibroses dont la cause est alcoolique et les stéatoses.

D'autre part, les outils de la présente invention sont plus fiables que ceux de l'art antérieur.

Ces outils sont : (1) un score diagnostique de la présence et de la sévérité de la fibrose, également appelé score diagnostique de la fibrose portale et septale (2) un moyen non-invasif de quantifier l'aire de fibrose, et (3) un moyen non-invasif de déterminer la dimension fractale indiquant le degré de distorsion du foie dû à la fibrose.

L'invention permet donc la détermination d'un score non-invasif diagnostique de la fibrose portale et septale (celle reflétée par le score Métavir) et cliniquement significative. Le score selon l'invention varie de 0 (fibrose minimale) à 1 (fibrose maximale) avec le seuil de référence fixé à 0,5 pour les scores de Métavir $F \geq 2$. Ce score est calculé à partir d'une référence subjective semi-quantitative de fibrose : le score Métavir. Le score Métavir est déterminé par un médecin anatomopathologiste après examen d'un fragment de foie au micoscope. L'échelle de ce score non-invasif est donc virtuelle car déformée par rapport à la mesure réelle (quoique elle aussi arbitraire et subjective) de fibrose représentée par un score de Métavir de 0 à 4. L'échelle est virtuelle car elle est générée par une formule mathématique et il n'y a pas d'unité de mesure, et cette échelle est déformée car il n'y a pas de proportionnalité directe (ou linéaire) entre les scores de Métavir et non-invasif. Cependant, ce score de 0 à 1 représente une mesure plus fine de la fibrose portale et septale car c'est une variable quantitative permettant des comparaisons plus fines. Deux exemples de résultant : un sujet pourra évoluer d'un score 0,14 à 0,28 alors qu'il est toujours en stade F0-F1 de Métavir et pourtant il a doublé son score de fibrose (progression de 100% en valeur relative). A l'inverse, lorsqu'un sujet évolue d'un score 0,48 à 0,52, il pourrait être déduit à tort qu'il est passé d'un stade F0-F1 à un stade F2-F3 (ou apparition d'une fibrose dite cliniquement significative) alors qu'en réalité la progression n'est que de 8% (en valeur relative - 0,48 par rapport à 0,52 ou [(0.52-0.48)/0.52] = 0,08 ou 8% - ou 4% - 0,52-0,48 = 0,04 - en valeur absolue et non cliniquement significative.

De plus, la présente invention permet non seulement de déterminer un score diagnostique mais aussi de quantifier l'aire de fibrose du foie. La mesure de l'aire de fibrose permet d'obtenir des résultats plus précis pour calculer le pourcentage du foie occupé par la fibrose que le score de fibrose F de Métavir actuellement utilisé. Une telle quantification n'était jusqu'alors possible dans aucune des méthodes décrites. Il s'agit d'un index (ou estimateur) de l'aire de fibrose variant de 2% à 55%, respectivement aire de fibrose minimale et maximale dans la population de malades de référence. Cet index est calculé avec une référence *quantitative* de fibrose. L'échelle de cet index est donc réelle car il est le reflet direct (non déformé) d'une mesure réelle objective et non arbitraire. C'est donc une mesure à la fois précise et parlante car estimant sans déformation une grandeur réelle. Deux exemples de résultats : un sujet pourra évoluer d'une aire de fibrose estimée de 8,2% à 16,4%. Un sujet avec cirrhose pourra régresser de 35% à 31% puis 27% et enfin 23% d'aire de fibrose estimée après arrêt de la cause ou sous traitement anti-fibrosant alors qu'en dépit d'une baisse régulière il est toujours au stade de cirrhose (F4).

De plus, la présente invention permet non seulement de déterminer un score diagnostique et de quantifier l'aire de fibrose du foie mais aussi de déterminer l'architecture du foie (dimension fractale). La mesure de l'architecture du foie permet d'obtenir des résultats plus précis pour évaluer le degré de distorsion du foie dû à la fibrose que le score de fibrose F de Métavir actuellement utilisé. Ce degré de distorsion du foie dû à la fibrose est la dimension fractale obtenue par analyse d'image qui repose sur plusieurs estimateurs dont la dimension de Kolgomorov (Moal F et al, 2002, Hepatology, vol.36, pages 840-9). Aucune des méthodes de l'art antérieur ne permet d'établir une mesure noninvasive de la dimension fractale par dosage de marqueurs sanguins.

En définitive, les inventeurs ont mis au point les scores suivants rassemblés dans le tableau 1 ci-dessous :

Tableau 1

| But du test : mesurer | Nom du test | Acronyme du test |
|---|---|---|
| *Dans une hépatite chronique virale :* | | |
| La présence d'une fibrose hépatique cliniquement significative | Score non-invasif de fibrose du foie | SNIFF |
| L'aire de fibrose hépatique | Score non-invasif de l'aire de fibrose du foie | SNIAFF |
| L'activité inflammatoire hépatique | Score non-invasif d'activité hépatique | SNIAH |
| *Dans une hépatite chronique alcoolique :* | | |
| La présence d'une fibrose hépatique cliniquement significative | Score non-invasif de fibrose du foie | SNIFFA |
| L'aire de fibrose hépatique | Score non-invasif de l'aire de fibrose du foie | SNIAFFA |
| *Dans une stéatose hépatique chronique :* | | |
| La présence d'une fibrose hépatique cliniquement significative | Score non-invasif de fibrose du foie | SNIFFSA |
| L'aire de fibrose hépatique | Score non-invasif de l'aire de fibrose du foie | SNIAFFSA |
| *Chez tout individu :* | | |
| La présence d'une fibrose hépatique cliniquement significative | Score non-invasif de dépistage de fibrose du foie | SNIDAFF |
| *Dans une hépatite chronique virale ou alcoolique :* | | |
| La présence d'une fibrose | Score non-invasif de | SNIFFAV |
| hépatique cliniquement significative | fibrose du foie | |
| L'aire de fibrose hépatique | Score non-invasif de l'aire de fibrose du foie | SNIAFFAV |
| La dimension fractale | Score non-invasif de dimension fractale de fibrose du foie | SNIDIFFA V |

La performance diagnostique est le pourcentage de sujets bien classés par rapport à la PBH. La performance diagnostique du score diagnostique de la présente invention augmente aux extrémités du score. Le score diagnostique SNIFF ne classe mal aucun malade avec hépatites virales pour F0 et F4 (et très peu pour F3). En d'autres termes, ce score SNIFF est très performant (100% de bonnes réponses) pour deux questions essentielles que se pose le clinicien : y a t-il un risque de classer à tort un sujet sans fibrose ou un sujet avec cirrhose ? La performance diagnostique d'un score SNIFF à 5 variables est de 90,8% pour 50,0% des malades avec les valeurs les plus basses et les plus hautes. Compte tenu des erreurs de la PBH surtout aux stades faibles (erreur de l'observateur) et élevés (erreur de l'échantillon) de fibrose, le taux d'erreur est donc proche de 0%.

Le but de l'invention est donc notamment de déterminer avec une plus grande précision que ne le permettent les outils de l'art antérieur, si un patient avec ou sans maladie du foie connue est atteint de fibrose, et la sévérité de l'atteinte du foie (degré lésionnel). Le test selon l'invention présente l'avantage de pouvoir être réalisé tous les 6 à 12 mois alors que la PBH ne peut être, éventuellement, répétée que tous les 3 à 5 ans selon les conférences de consensus.

La méthode selon l'invention consiste à combiner et à mesurer différents marqueurs de fibrose directs associés à des marqueurs indirects pris dans une combinaison spécifique, lesdits marqueurs étant appelés variables. Ces variables sont mesurées dans un échantillon d'un sujet. Le choix de ces variables est déterminé par la meilleure performance globale de la combinaison de variables obtenue par analyse statistique de différents modèles mathématiques, chacune apportant une information statistiquement significative et indépendante des autres. Autrement dit, il s'agit de la meilleure performance pour le moindre nombre de variables. Ceci signifie que toute nouvelle variable dans le modèle mathématique apporte une information inventive (ou gain de performance diagnostique) par rapport à une combinaison plus restreinte ayant déjà pu faire l'objet d'une publication.

Par « échantillon », on entend dans le cadre de la présente invention un échantillon prélevé sur un sujet préalablement à toute analyse. Cet échantillon peut être un milieu biologique tel que du sang, du sérum, du plasma, de l'urine ou de la salive dudit sujet ou une ou plusieurs cellules dudit sujet telles qu'un biopsie tissulaire et, plus particulièrement, une biopsie hépatique.

Par « pathologie hépatique », on entend une pathologie hépatique choisie parmi une fibrose hépatique chronique d'origine virale, fibrose hépatique chronique d'origine alcoolique et une stéatose chronique hépatique chronique

Par « sujet », on entend dans le cadre de la présente invention un homme, une femme ou un animal, jeunes ou adultes, sains ou susceptibles d'être atteints ou atteints par une pathologie hépatique telle qu'une fibrose hépatique chronique d'origine virale, fibrose hépatique chronique d'origine alcoolique ou une stéatose chronique hépatique ou par toute autre pathologie, le sujet atteint pouvant ou non recevoir un traitement curatif contre cette pathologie hépatique.

La présente invention concerne donc une méthode pour diagnostiquer la présence et/ou la sévérité d'une pathologie hépatique et/ou pour suivre l'efficacité d'un traitement curatif contre une pathologie hépatique chez un sujet comprenant l'établissement d'au moins un score diagnostique non-invasif, en particulier d'un score diagnostique de la fibrose portale et septale et/ou d'un estimateur non-invasif de l'aire de fibrose, et/ou d'un estimateur non-invasif de la dimension fractale, par la mise en oeuvre des étapes suivantes :

a) pour la détermination de l'aire de fibrose ou de la dimension fractale,
mesurer dans un échantillon dudit sujet au moins une variable choisie dans le groupe constitué par $\alpha$-2 macroglobuline (A2M), acide hyaluronique (AH ou hyaluronate), apoliprotéine A1 (ApoAI), propeptide N-terminal du procollagène de type III (P3P), gamma-glutamyltranspeptidase (GGT), bilirubine, gamma-globulines (GLB), plaquettes (PLQ), taux de prothrombine (TP), aspartate amino-transférase (ASAT), alanine amino-transférase (ALAT), urée, sodium (NA), glycémie, triglycérides, albumine (ALB), phosphatases alcalines (PAL), YKL-40 (human cartilage glycoprotein 39), Tissue inhibitor of matrix metalloproteinase 1 (TIMP-1), Matrix metalloproteinase 2 (MMP-2), Ferritine,

a') pour l'établissement d'un score diagnostique de la fibrose portale et septale, mesurer dans un échantillon dudit sujet au moins trois variables choisies dans le groupe constitué par $\alpha$-2 macroglobuline (A2M), acide hyaluronique (AH ou hyaluronate), apoliprotéine A1 (ApoAI), propeptide N-terminal du procollagène de type III (P3P), gamma-glutamyltranspeptidase (GGT), bilirubine, gamma-globulines (GLB), plaquettes (PLQ), taux de prothrombine (TP), aspartate amino-transférase (ASAT), alanine amino-transférase (ALAT), urée, sodium (NA), glycémie, triglycérides, albumine (ALB), phosphatases alcalines (PAL), YKL-40 (human cartilage glycoprotein 39), Tissue inhibitor of matrix metalloproteinase 1 (TIMP-1), Matrix metalloproteinase 2 (MMP-2), Ferritine ; l'une au moins des trois variables étant choisie parmi le groupe constitué par plaquettes (PLQ) et taux de prothrombine (TP) ; dans le cas où il est mesuré exactement trois variables, ces trois variables ne peuvent pas être ensemble plaquettes (PLQ), taux de prothrombine (TP) et bilirubine ; de préférence, les au moins trois variables choisies ne comprennent pas ensemble $\alpha$-2 macroglobuline (A2M), acide hyaluronique (AH ou hyaluronate) et Tissue inhibitor of matrix metalloproteinase 1 (TIMP-1),

b) éventuellement, recueillir au moins une variable clinique caractérisant ledit sujet ;
pour le score diagnostique de la fibrose portale et septale, les étapes a') et b) ci-dessus étant telles que au moins 4 variables sont mesurées ou recueillies,

c) combiner, dans une fonction logistique ou linéaire, lesdites variables afin d'obtenir un score diagnostique de la fibrose portale et septale et/ou d'un estimateur diagnostique de l'aire de fibrose, et/ou d'un estimateur diagnostique de la dimension fractale ;

d) diagnostiquer la présence et/ou la sévérité de ladite pathologie et/ou l'efficacité dudit traitement à partir du score obtenu lors de la combinaison de l'étape (c).

Suivant un premier mode de réalisation de l'invention, à l'étape a', les au moins trois variables sont choisies dans le groupe constitué par $\alpha$-2 macroglobuline (A2M), apoliprotéine A1 (ApoAI), propeptide N-terminal du procollagène de type III (P3P), gamma-glutamyltranspeptidase (GGT), bilirubine, gamma-globulines (GLB), plaquettes (PLQ), taux de prothrombine (TP), aspartate amino-transférase (ASAT), alanine amino-transférase (ALAT), urée, sodium (NA), glycémie, triglycérides, albumine (ALB), phosphatases alcalines (PAL), YKL-40 (human cartilage glycoprotein 39), Tissue inhibitor of matrix metalloproteinase 1 (TIMP-1), Matrix metalloproteinase 2 (MMP-2), Ferritine ; l'une au moins des trois variables étant choisie parmi le groupe constitué par plaquettes (PLQ) et taux de prothrombine (TP) ; dans le cas où il est mesuré exactement trois variables, ces trois variables ne peuvent pas être ensemble plaquettes (PLQ), taux de prothrombine (TP) et bilirubine ; de préférence, les au moins trois variables choisies ne comprennent pas ensemble $\alpha$-2 macroglobuline (A2M), acide hyaluronique (AH ou hyaluronate) et Tissue inhibitor of matrix metalloproteinase 1 (TIMP-1).

Suivant un second mode de réalisation de l'invention, à l'étape a', les au moins trois variables sont choisies dans le groupe constitué par $\alpha$-2 macroglobuline (A2M), acide hyaluronique (AH ou hyaluronate), apoliprotéine A1 (ApoAI), propeptide N-terminal du procollagène de type III (P3P), gamma-glutamyltranspeptidase (GGT), bilirubine, gamma-globulines (GLB), plaquettes (PLQ), taux de prothrombine (TP), aspartate amino-transférase (ASAT), alanine amino-transférase (ALAT), urée, sodium (NA), glycémie, triglycérides, albumine (ALB), phosphatases alcalines (PAL), YKL-40 (human cartilage glycoprotein 39), Matrix metalloproteinase 2 (MMP-2), Ferritine ; l'une au moins des trois variables étant choisie parmi le groupe constitué par plaquettes (PLQ) et taux de prothrombine (TP) ; dans le cas où il est mesuré exactement trois variables, ces trois variables ne peuvent pas être ensemble plaquettes (PLQ), taux de prothrombine (TP) et bilirubine ; de préférence, les au moins trois variables choisies ne comprennent pas ensemble $\alpha$-2 macroglobuline (A2M), acide hyaluronique (AH ou hyaluronate) et Tissue inhibitor of matrix metalloproteinase 1 (TIMP-1).

L'invention concerne également un test de diagnostic de la fibrose hépatique, qui met en oeuvre la méthode de l'invention. Par diagnostic au sens de la présente invention, on entend l'établissement de la présence d'une fibrose et/ou de son stade d'évolution. Pour établir le diagnostic, la spécificité du test ou de la méthode employée est généralement privilégiée.

De façon avantageuse, les variables cliniques caractérisant le sujet sont choisies parmi le poids corporel (poids), l'index de masse corporelle (IMC ou BMI en anglais soit le rapport poids /(taille ou hauteur)$^2$, l'âge (âge) à la date du recueil de l'échantillon et la cause. Par « cause » (ou étiologie), on entend la cause alcoolique ou virale. Par conséquent, il est clair pour l'homme du métier que la variable clinique « cause » ne pourra être utilisée qu'à partir du moment où une pathologie hépatique telle qu'une fibrose hépatique chronique d'origine virale ou fibrose hépatique chronique d'origine alcoolique aura été préalablement diagnostiquée.

Dans le procédé de l'invention, préalablement à l'étape (c), les variables mesurées à l'étape (a) ou (a') et les variables recueillies à l'étape (b) peuvent être combinées les unes aux autres. Par conséquent, on peut utiliser dans la fonction logistique mise en oeuvre dans le cadre de l'invention soit des « variables natives » également appelées « variables isolées ou simples » qui sont des variables n'ayant subi aucune modification avant l'introduction dans la fonction logistique, soit des « variables combinatoires » qui sont des combinaisons arithmétiques de variables isolées entre elles. A titre d'exemples de variables combinatoires utiles dans le cadre de la présente invention et de façon non exhaustive, on trouve :

- GAPRI = ((GGT/45) / PLQ) * 100
- GLOPRI = (GLB / PLQ) * 100
- GLOTRI = (GLB / TP) * 100
- HYAPRI = (AH / PLQ) * 100
- HYATRI = (AH / TP) * 100
- AMPRI = (A2M / PLQ) * 100
- AMTRI = (A2M / TP) * 100
- HYAMTRI = (AH * A2M) / (TP * 100)
- HYAMPRI = (AH * A2M) / (A2M * 100)= AH/100
- HAMPRI = (AH * A2M) / (PLQ * 100)
- HYAMPTRI = (AH * A2M) / (PLQ * TP)
- GHAMPRI = (GLB * AH * A2M) / (PLQ * 1000)
- GHAMTRI = (GLB * AH * A2M) / (TP * 1000)
- GHAMPTRI = (GLB * AH * A2M) / (PLQ * TP * 10)

Le sigle de ces variables combinatoires utilise l'abréviation des variables isolées (simples) en préfixe et le suffixe RI signifie « ratio index ».

A noter qu'un score différent, mais similaire dans son principe, appelé APRI (= ASAT/PLQ) a été publié (Wai et al, Hepatology, 2003, vol 38, pages 518-526). Le rapport ASAT/ALAT appelé ci-après RAT fait également partie de l'état de la technique.

Selon la présente invention, on appelle score non invasif de fibrose du foie (acronyme : SNIFF) un score composé d'une combinaison de marqueurs de préférence sanguins, variant de 0 à 1, estimateur du score de type Métavir F pour les maladies hépatiques d'origine virale (SNIFF) ou alcoolique (SNIFFA) ou les deux causes (SNIFFAV) ou stéatosique (SNIFFSA). On appelle score non invasif de l'aire de fibrose du foie (acronyme : SNIAFF) un score composé d'une combinaison de marqueurs de préférence sanguins variant, dans la majorité des cas, de 5 à 55%. C'est un estimateur de l'aire de fibrose du foie pour les maladies hépatiques d'origine virale (SNIAFF) ou alcoolique (SNIAFFA) ou les deux causes (SNIAFFAV) ou stéatosique (SNIAFFSA).

La sévérité d'une pathologie hépatique est l'évaluation du degré de fibrose dans le foie.

Dans l'étape (a') de la méthode de l'invention, on mesure dans un échantillon dudit sujet au moins 3 variables, de préférence 4, 5, 6 ou 7 variables.

Les mesures effectuées à l'étape (a) ou (a') de la méthode de l'invention sont des mesures visant soit à quantifier la variable (cas pour A2M, AH, bilirubine, PLQ, TP, Urée, NA, glycémie, triglycérides, ALB, P3P), soit à quantifier l'activité enzymatique de la variable (cas pour GGT, ASAT, ALAT, PAL). L'homme du métier connaît différentes méthodes directes ou indirectes permettant de quantifier une substance donnée ou une protéine ou son activité enzymatique. Ces méthodes peuvent mettre en oeuvre un ou plusieurs anticorps monoclonaux ou polyclonaux reconnaissant ladite protéine dans des techniques d'immunoessais (radio-immunoessais ou RIA, tests ELISA, Western Blot, etc...), l'analyse des taux d'ARNm de ladite protéine en utilisant des techniques du type Northern Blot, Slot Blot ou PCR, des techniques comme une HPLC éventuellement combinée à une spectrophotométrie de masse, etc... Les dosages d'activité des protéines citées précédemment mettent en oeuvre des dosages effectués sur au moins un substrat spécifique de chacune de ces protéines. La demande de brevet internationale WO03/073822 liste des méthodes utilisables pour quantifier α-2 macroglobuline (A2M) et acide hyaluronique (AH ou hyaluronate).

A titre d'exemples et de façon non exhaustive, une liste préférée de dosage ou de kits commerciaux utilisables pour les mesures effectuées à l'étape (a) ou (a') de la méthode objet de la présente invention sur des échantillons sanguins est donnée ci-après :

- Taux de prothrombine : le temps de Quick (TQ) est déterminé en ajoutant au plasma de la thromboplastine calcique (par exemple Neoplastin CI plus, Diagnostica Stago, Asnières, France) et le temps de coagulation est mesuré en secondes. Pour obtenir le taux de prothrombine (TP), une droite d'étalonnage est faite à partir de différentes dilutions d'un pool de plasma normaux estimé à 100%. Les résultas obtenus pour les plasmas de patients sont exprimés en pourcentage par rapport au pool de plasma normaux. La valeur supérieure du TP n'est pas limitée et peut dépasser 100%.
- A2M : son dosage est effectué par immunonéphélométrie laser utilisant par exemple un analyseur néphélomètre Behring. Le réactif peut être un anti-sérum de lapin contre l'A2M humaine.
- AH : les concentrations sériques sont déterminées avec un ELISA (par exemple : Corgenix, Inc. Biogenic SA 34130 Mauguio France) qui utilise des protéines spécifiques fixant l'AH isolées à partir de cartilage bovin.
- P3P . les concentrations sériques sont déterminées avec un RIA (par exemple : RIA-gnost PIIIP kit, Hoechst, Tokyo, Japan) utilisant un anticorps monoclonal murin dirigé contre le PIIINP de peau bovine.
- PLQ . les échantillons sanguins sont collectés dans des vacutainers contenant de l'EDTA (ethylenediamine acide éthylène diamine tétraacétique) (par exemple Becton Dickinson, France) et peuvent être analysés sur un compteur Advia 120 (Bayer Diagnostic).
- Urée : dosage par exemple par « Kinectic UV assay for urea » (Roche Diagnostics).
- GGT : dosage par exemple par « Gamma-glutamyltranferase assay standardized against Szasz » (Roche Diagnostics).
- Bilirubine : dosage par exemple par « Bilirubin assay » (Jendrassik-Grof method) (Roche Diagnostics).
- PAL : dosage par exemple par « ALP IFCC » (Roche Diagnostics).
- ALAT : dosage par exemple par « ALT IFCC » (Roche Diagnostics).
- ASAT : dosage par exemple par « AST IFCC » (Roche Diagnostics).
- Sodium : dosage par exemple par « Sodium ion selective electrode » (Roche Diagnostics).
- Glycémie : dosage par exemple par « Glucose GOD-PAP » (Roche Diagnostics).
- Triglycérides : dosage par exemple par
« triglycerides GPO-PAP » (Roche Diagnostics).
- Urée, GGT, bilirubine, phosphatases alcalines, sodium, glycémie, ALAT et ASAT peuvent être dosés sur un analyseur, par exemple, Hitachi 917, Roche Diagnostics GmbH, D-68298 Mannheim, Germany.
- Gamma-globulines, albumine et alpha-2 globulines :

    dosage sur électrophorèse des protéines, par exemple :

        Capillary electrophoresis (Capillarys), SEBIA 23, rue M Robespierre, 92130 Issy Les Moulineaux, France.

- ApoAI : dosage par exemple par « Determination of apolipoprotein A-1 » (Dade Behring) avec analyseur, par exemple : BN2 Dade Behring Marburg GmbH, Emil von Behring Str. 76, D-35041 Marburg, Germany.
- TIMP1 : dosage par exemple par TIMP1- Elisa, Amersham.
- MMP2 : dosage par exemple par MMP2- Elisa, Amersham.
- YKL-40 : dosage par exemple par YKL-40 Biometra,, YKL-40/8020, Quidel Corporation.
- PIIIP : dosage par exemple par PIIIP RIA Kit, OCFKO7-PIIIP, cis bio international.

Pour les variables mesurées à l'étape (a) ou (a') de la méthode objet de la présente invention, les valeurs obtenues sont exprimées en :

- mg/dl pour α-2 macroglobuline (A2M),
- μg/l pour acide hyaluronique (AH ou hyaluronate),
- g/l pour apoliprotéine A1 (ApoAI)**,

- U/ml pour propeptide N-terminal du procollagène de type III (P3P)**,
- UI/l pour gamma-glutamyltranspeptidase (GGT),
- μmol/l pour bilirubine,
- g/l pour gamma-globulines (GLB)*,
- Giga/l pour plaquettes (PLQ),
- % pour taux de prothrombine (TP),
- UI/l pour aspartate amino-transférases (ASAT),
- UI/l pour alanine amino-transférases (ALAT),
- mmol/l triglycérides*,
- mmol/l pour urée*,
- mmol/l pour sodium (NA),
- mmol/l pour glycémie*,
- g/l pour albumine (ALB)*,
- UI/l pour phosphatases alcalines (PAL),
- ng/ml pour TIMP1,
- ng/ml pour MMP2,
- ng/ml pour YKL-40,
- U/ml pour PIIIP,
- μg/l pour ferritine.

Les variables cliniques recueillies à l'étape (b) de la méthode objet de la présente invention sont exprimées en

- kg pour le poids corporel (poids) à la date du recueil de l'échantillon,
- ans pour l'âge (âge)* à la date du recueil de l'échantillon,
- kg/m$^2$ dans le BMI ou IMC* : kg pour le poids corporel, m (mètre) pour la hauteur corporelle,
- code 1 pour la cause alcoolique et 2 pour la cause virale.

Les variables repérées par une astérisque (*) sont exprimées avec une (*) ou deux (**) décimales, les autres le sont sans décimale.

De façon avantageuse, l'échantillon du sujet utilisé à l'étape (a) ou (a') de la méthode objet de la présente invention est un milieu biologique tel que du sang, du sérum, du plasma, de l'urine ou de la salive dudit sujet ou une ou plusieurs cellules dudit sujet telles qu'un biopsie tissulaire et, plus particulièrement, une biopsie hépatique. On peut envisager, dans le cadre de la présente invention que les différentes variables mesurées à l'étape (a) ou (a') sont mesurées dans des échantillons différents du patient. A titre d'exemples et de façon non exhaustive, une variable est mesurée dans l'urine du sujet alors que trois autres sont mesurées dans le sang du même sujet, les deux prélèvements (sang et urine) étant effectués dans un laps de temps relativement court. Toutefois et de façon particulièrement préférée, l'échantillon du sujet utilisé à l'étape (a) ou (a') de la méthode objet de la présente invention est un prélèvement sanguin effectué sur le sujet avant toute mesure.

Suivant un premier mode de réalisation de la présente invention, on combine, à l'étape (c) de la méthode objet de la présente invention, les variables α-2 macroglobuline (A2M) et taux de prothrombine (TP) et au moins deux variables choisies parmi plaquettes (PLQ), aspartate amino-transférase (ASAT), urée, acide hyaluronique (AH) et âge. De façon avantageuse, le score obtenu est un score non invasif de la fibrose du foie d'origine virale à au moins 4 variables.

Parmi les scores préférés susceptibles d'être obtenus dans ce premier mode de réalisation, on préfère les scores pour lesquels on combine à l'étape (c) :

- α-2 macroglobuline (A2M), taux de prothrombine (TP), acide hyaluronique (AH) et âge (score appelé SNIFF 4a) ;
- α-2 macroglobuline (A2M), taux de prothrombine (TP), aspartate amino-transférase (ASAT) et âge (score appelé SNIFF 4b) ;
- α-2 macroglobuline (A2M), taux de prothrombine (TP), plaquettes (PLQ), aspartate amino-transférase (ASAT) et âge (score appelé SNIFF 5) ;
- α-2 macroglobuline (A2M), taux de prothrombine (TP), plaquettes (PLQ), aspartate amino-transférase (ASAT), urée et acide hyaluronique (AH) (score appelé SNIFF 6) ;
- α-2 macroglobuline (A2M), taux de prothrombine (TP), plaquettes (PLQ), aspartate amino-transférase (ASAT), urée, acide hyaluronique (AH) et âge (score appelé SNIFF 7).

Le score pouvant être ainsi obtenu est un score non invasif de fibrose du foie d'origine virale appelé SNIFF, qui donne un score de 0 à 1 estimateur du score de type Métavir F mettant en oeuvre de 4 à 7 variables.

Dans un second mode de réalisation de la présente invention, on combine à l'étape (c), outre la variable taux de prothrombine (TP), au moins trois variables choisies parmi aspartate amino-transférase (ASAT), alanine amino-transférase (ALAT) et phosphatases alcalines (PAL), âge, acide hyaluronique (AH ou hyaluronate) et α-2 macroglobuline (A2M). Le score pouvant être ainsi obtenu est un score non invasif de fibrose du foie d'origine alcoolique appelé **SNIFFA.**

Parmi les scores préférés susceptibles d'être obtenus dans ce deuxième mode de réalisation, on préfère les scores pour lesquels on combine à l'étape (c) :

- taux de prothrombine (TP), aspartate amino-transférase (ASAT), alanine amino-transférase (ALAT) et phosphatases alcalines (PAL) (score appelé SNIFFA 4b),
- taux de prothrombine (TP), âge, acide hyaluronique (AH ou hyaluronate) et α-2 macroglobuline (A2M) (score appelé SNIFFA 4c).

Suivant un troisième mode de réalisation de l'invention, on combine à l'étape (c) de la méthode au moins les 4 variables suivantes : acide hyaluronique (AH ou hyaluronate), gamma-glutamyltranspeptidase (GGT), bilirubine et plaquettes (PLQ). Le score ainsi obtenu est un score non invasif (appelé **SNIAFF** à au moins 4 variables) estimateur de l'aire de fibrose du foie variant, dans la majorité des cas, de 5 à 35%. De façon préférée, on combine, à l'étape (c), en plus des quatre variables décrites précédemment, au moins une et de préférence au moins deux variables et de manière encore préférée, au moins quatre variables choisies parmi α-2 macroglobuline (A2M), urée, apoliprotéine A1 (ApoAl) et gamma-globulines (GLB).

Parmi les scores préférés susceptibles d'être obtenus dans ce troisième mode de réalisation, on préfère les scores pour lesquels on combine à l'étape (c) :

- acide hyaluronique (AH ou hyaluronate), gamma-glutamyltranspeptidase (GGT), bilirubine, plaquettes (PLQ) et apoliprotéine A1 (ApoAl) (score appelé SNIAFF 5)
- acide hyaluronique (AH ou hyaluronate), gamma-glutamyltranspeptidase (GGT), bilirubine, plaquettes (PLQ), α-2 macroglobuline (A2M) et urée (score appelé SNIAFF 6a)
- acide hyaluronique (AH ou hyaluronate), gamma-glutamyltranspeptidase (GGT), bilirubine, plaquettes (PLQ) , urée et gamma-globulines (GLB) (score appelé SNIAFF 6b).

Dans un quatrième mode de réalisation de la présente invention, un score appelé **SNIDAFF** qui est un score non invasif de dépistage de la fibrose du foie à partir de variables usuelles pour les hépatopathies alcooliques et virales variant de 0 à 1 peut être obtenu. Par dépistage au sens de la présente invention, il faut entendre la recherche de la présence d'une fibrose quelque soit son stade, soit chez des patients sans maladie du foie connue, soit chez des patients ayant une maladie chronique du foie connue. Pour le dépistage, la sensibilité du test est un critère particulièrement important.

Le score SNIDAFF peut être avantageusement obtenu en combinant à l'étape (c) de la méthode de la présente invention, au moins les quatre variables suivantes : plaquettes (PLQ), taux de prothrombine (TP), aspartate amino-transférase (ASAT) et âge. De façon préférée, on combine, à l'étape (c), en plus des quatre variables décrites précédemment, au moins une et de préférence au moins deux variables choisies parmi phosphatases alcalines (PAL), α-2 macroglobuline (A2M) et urée.

Ainsi, parmi les scores préférés susceptibles d'être obtenus dans ce quatrième mode de réalisation, on préfère les scores pour lesquels on combine à l'étape (c) :

- plaquettes (PLQ), taux de prothrombine (TP), aspartate amino-transférase (ASAT), âge, phosphatases alcalines (PAL) et α-2 macroglobuline (score appelé SNIDAFF 6a) ;
- plaquettes (PLQ), taux de prothrombine (TP), aspartate amino-transférase (ASAT), âge, phosphatases alcalines (PAL) et urée (score appelé SNIDAFF 6b).

Dans un cinquième mode de réalisation de la présente invention, un score appelé **SNIFFSA** qui est un score non invasif de la fibrose du foie pour les hépatopathies stéatosiques variant de 0 à 1 peut être obtenu. Le score SNIFFSA peut être avantageusement obtenu en combinant à l'étape (c) de la méthode de la présente invention, outre la variable taux de prothrombine (TP), au moins trois variables choisies parmi aspartate amino-transférase (ASAT), triglycérides, âge et glycémie.

Parmi les scores préférés susceptibles d'être obtenus dans ce cinquième mode de réalisation, on préfère les scores pour lesquels on combine à l'étape (c) :

- taux de prothrombine (TP), aspartate amino-transférase (ASAT), âge et glycémie (score appelé SNIFFSA 4a),
- taux de prothrombine (TP), triglycérides, âge et glycémie (score appelé SNIFFSA 4b).

Dans un sixième mode de réalisation de la présente invention, le score appelé **SNIFFAV** qui est un score non invasif de la fibrose du foie pour les hépatopathies virales ou alcooliques variant de 0 à 1 peut être obtenu. Le score SNIFFAV peut être avantageusement obtenu en combinant à l'étape (c) de la méthode de la présente invention, au moins cinq des 6 variables suivantes : $\alpha$-2 macroglobuline (A2M), plaquettes (PLQ), taux de prothrombine (TP), urée, acide hyaluronique (AH ou hyaluronate) ou cause.

Parmi les scores préférés susceptibles d'être obtenus dans ce sixième mode de réalisation, on préfère les scores pour lesquels on combine à l'étape (c) :

- $\alpha$-2 macroglobuline (A2M), plaquettes (PLQ), taux de prothrombine (TP), urée, acide hyaluronique (AH ou hyaluronate) (score appelé SNIFFAV 5).
- $\alpha$-2 macroglobuline (A2M), plaquettes (PLQ), taux de prothrombine (TP), urée, acide hyaluronique (AH ou hyaluronate) et cause (score appelé SNIFFAV 6).

Dans un septième mode de réalisation de la présente invention, le score appelé **SNIAFFAV** qui est un score non invasif estimateur de l'aire de fibrose du foie pour les hépatopathies virales ou alcooliques variant, dans la majorité des cas, de 5 à 55%, peut être obtenu. Le score SNIAFFAV peut être avantageusement obtenu en combinant à l'étape (c) de la méthode de la présente invention, outre la variable taux de prothrombine (TP), au moins trois, de préférence au moins quatre ou, de façon encore préférée, cinq, six ou sept variables choisies parmi plaquettes (PLQ), urée, acide hyaluronique (AH ou hyaluronate), bilirubine, $\alpha$-2 macroglobuline (A2M), gamma-glutamyltranspeptidase (GGT), gamma-globulines (GLB), aspartate amino-transférase (ASAT) et cause.

Ainsi, parmi les scores préférés susceptibles d'être obtenus dans ce septième mode de réalisation, on préfère les scores pour lesquels on combine à l'étape (c) :

- taux de prothrombine (TP), acide hyaluronique (AH ou hyaluronate), bilirubine et $\alpha$-2 macroglobuline (A2M) (score appelé SNIAFFAV 4),
- taux de prothrombine (TP), plaquettes (PLQ), urée, acide hyaluronique (AH ou hyaluronate) et cause (score appelé SNIAFFAV 5),
- taux de prothrombine (TP), urée, acide hyaluronique (AH ou hyaluronate), bilirubine et $\alpha$-2 macroglobuline (A2M) (score appelé SNIAFFAV 5b),
- taux de prothrombine (TP), acide hyaluronique (AH ou hyaluronate), bilirubine, $\alpha$-2 macroglobuline (A2M) et cause (score appelé SNIAFFAV 5c),
- taux de prothrombine (TP), plaquettes (PLQ), acide hyaluronique (AH ou hyaluronate), bilirubine, $\alpha$-2 macroglobuline (A2M), gamma-glutamyltranspeptidase (GGT), gamma-globulines (GLB) et aspartate amino-transférase (ASAT) (score appelé SNIAFFAV 8).

Dans un huitième mode de réalisation de la présente invention, le score appelé SNIDIFFAV qui est un score non invasif estimateur de la dimension fractale de la fibrose du foie pour les hépatopathies virales ou alcooliques variant, dans la majorité des cas de 0,7 à 1,3 peut être obtenu. Le score **SNIDIFFAV** peut être avantageusement obtenu en combinant à l'étape (c) de la méthode de la présente invention, au moins quatre des 5 variables suivantes : $\alpha$-2 macroglobuline (A2M), albumine (ALB), taux de prothrombine (TP), acide hyaluronique (AH ou hyaluronate), alanine amino-transférase (ALAT), aspartate amino-transférase (ASAT) et âge.

Parmi les scores préférés susceptibles d'être obtenus dans ce huitième mode de réalisation, on préfère les scores pour lesquels on combine à l'étape (c) :

- $\alpha$-2 macroglobuline (A2M), taux de prothrombine (TP), albumine (ALB) et âge (score appelé SNIDIFFAV 4a),
- $\alpha$-2 macroglobuline (A2M), taux de prothrombine (TP), albumine (ALB) et acide hyaluronique (AH ou hyaluronate) (score appelé SNIDIFFAV 4b),
- $\alpha$-2 macroglobuline (A2M), albumine (ALB), taux de prothrombine (TP), alanine amino-transférase (ALAT), aspartate amino-transférase (ASAT) et âge (score appelé SNIDIFFAV 6).

Dans un neuvième mode de réalisation de la présente invention, le score appelé **SNIAFFA** qui est un score non invasif estimateur de l'aire de fibrose du foie pour les hépatopathies alcooliques variant, dans la majorité des cas de 5 à 55% peut être obtenu. Le score SNIAFFA peut être avantageusement obtenu en combinant à l'étape (c) de la méthode de la présente invention, outre la variable taux de prothrombine (TP) au moins trois variables choisies parmi $\alpha$-2 macroglobuline (A2M), acide hyaluronique (AH ou hyaluronate), plaquettes (PLQ) et poids du sujet.

Parmi les scores préférés susceptibles d'être obtenus dans ce neuvième mode de réalisation, on préfère les scores pour lesquels on combine à l'étape (c) :

- taux de prothrombine (TP), α-2 macroglobuline (A2M), acide hyaluronique (AH ou hyaluronate) et poids du sujet (scores appelés SNIAFFA 4a et SNIAFFA 4b),
- taux de prothrombine (TP), α-2 macroglobuline (A2M), acide hyaluronique (AH ou hyaluronate) et plaquettes (PLQ) (score appelé SNIAFFA 4c).

Dans un dixième mode de réalisation de la présente invention, le score appelé **SNIAFFSA** qui est un score non invasif estimateur de l'aire de fibrose du foie pour les hépatopathies stéatosiques variant, dans la majorité des cas de 5 à 35% peut être obtenu. Le score SNIAFFSA peut être avantageusement obtenu en combinant à l'étape (c) de la méthode de la présente invention, outre les trois variables taux de prothrombine (TP), gammaglobulines (GLB) et poids au moins une variable, de préférence, au moins deux variables choisies parmi acide hyaluronique (AH ou hyaluronate), plaquettes (PLQ), âge et IMC (BMI) du sujet.

Parmi les scores préférés susceptibles d'être obtenus dans ce dixième mode de réalisation, on préfère les scores pour lesquels on combine à l'étape (c) :

- taux de prothrombine (TP), gammaglobulines (GLB), poids et âge (score appelé SNIAFFSA 4),
- taux de prothrombine (TP), gammaglobulines (GLB), poids, acide hyaluronique (AH ou hyaluronate), plaquettes (PLQ) et IMC (BMI) (score appelé SNIAFFSA 6).

En variante, la présente invention concerne également une méthode pour diagnostiquer la présence et/ou indiquer la sévérité d'une pathologie hépatique et/ou pour suivre l'efficacité d'un traitement curatif contre une pathologie hépatique chez un sujet comprenant les étapes suivantes :

a') mesurer dans un échantillon dudit sujet au moins une variable choisie dans le groupe constitué par α-2 macroglobuline (A2M), acide hyaluronique (AH ou hyaluronate), apoliprotéine A1 (ApoA1) , gamma-glutamyltranspeptidase (GGT), bilirubine, gamma-globulines (GLB), plaquettes (PLQ), taux de prothrombine (TP), aspartate amino-transférase (ASAT), alanine amino-transférase (ALAT), urée, sodium (NA), triglycérides, glycémie, albumine (ALB) et phosphatases alcalines (PAL), YKL-40 (human cartilage glycoprotein 39), Tissue inhibitor of matrix metalloproteinase 1 (TIMP-1), Matrix metalloproteinase 2 (MMP-2), Ferritine, ;
b') éventuellement recueillir au moins une variable clinique caractérisant ledit sujet ;
c') combiner, dans une fonction logistique ou linéaire, la ou les variables mesurées en (a') et éventuellement les variables recueillies en (b') afin d'obtenir un score ;
d') diagnostiquer la présence et/ou la sévérité de ladite pathologie à partir du score obtenu lors de la combinaison de l'étape (c').

Les caractéristiques des étapes (a), (b), (c) et (d) décrites précédemment (échantillon, dosage des variables, unité des variables) s'appliquent mutatis mutandis aux étapes (a'), (b'), (c') et (d').

Suivant une première forme de mise en oeuvre de cette variante de l'invention, les deux variables suivantes : α-2 macroglobuline (A2M) et acide hyaluronique (AH ou hyaluronate) peuvent être mesurées à l'étape (a') pour obtenir le score appelé SNIAFFA 2 qui est un score non invasif estimateur de l'aire de fibrose du foie pour les hépatopathies alcooliques variant, dans la majorité des cas de 5 à 55%.

A ces deux variables, peut être combinée au moins une et de préférence au moins deux variables choisies parmi le poids du sujet et propeptide N-terminal du procollagène de type III (P3P).

Parmi les scores préférés susceptibles d'être obtenus dans la première forme de mise en oeuvre de cette variante de l'invention, on préfère les scores pour lesquels on combine à l'étape (c) :

- α-2 macroglobuline (A2M), acide hyaluronique (AH ou hyaluronate) et poids du sujet permettant ainsi d'obtenir le score SNIAFFA 3 basé sur 3 variables,
- α-2 macroglobuline (A2M), acide hyaluronique (AH ou hyaluronate), poids du sujet et propeptide N-terminal du procollagène de type III (P3P) (score appelé SNIAFFA 4).

Suivant une deuxième forme de mise en oeuvre de cette variante de l'invention, la variable alanine amino-transférase (ALAT) peut être mesurée à l'étape (a') de la méthode de l'invention pour obtenir le score appelé **SNIAH** qui est un score non invasif estimateur de l'activité nécrotico-inflammatoire du foie pour les hépatopathies virales.

Suivant une troisième forme de mise en oeuvre de cette variante de l'invention, on mesure à l'étape (a') de l'invention les trois variables biologiques suivantes : taux de prothrombine (TP), alanine amino-transférase (ALAT) et phosphatases alcalines (PAL). Ces trois variables combinées ensemble dans l'étape (c') de la présente invention permettent d'obtenir le score SNIFFA 3 (score non invasif de fibrose du foie d'origine alcoolique à 3 variables).

De plus, de façon alternative, des scores non invasifs de fibrose du foie appelés SNIFFA d'origine alcoolique mettant en oeuvre quatre variables peuvent être utilisées. Ainsi, le score SNIFFA 4 mettant en oeuvre quatre variables est déterminé en combinant à l'étape (c') les variables suivantes : $\alpha$-2 macroglobuline (A2M), âge, acide hyaluronique (AH ou hyaluronate) et alanine amino-transférase (ALAT) permettant d'obtenir le score SNIFFA 4a.

Les scores SNIFF, SNIFFA, SNIFFSA, SNIAH, SNIDAFF et SNIFFAV (ou variable dépendante) sont prédits par une combinaison de marqueurs biologiques ou cliniques (ou variables indépendantes). Ces combinaisons (ou modèles) ont été obtenues par la méthode statistique appelée régression logistique binaire avec la procédure suivante :

Dans un premier temps, les variables indépendantes ont été testées en analyse univariée.

Dans un deuxième temps, les variables indépendantes significatives en analyse univariée ont été testées en analyse multivariée par régression logistique binaire avec sélection pas à pas ascendante ou descendante.

La régression logistique produit la formule de chaque score sous la forme :

$$\texttt{score = a}_0 \texttt{ + a}_1 \texttt{ x}_1 \texttt{ + a}_2 \texttt{ x}_2 \texttt{ + …}$$

où les coefficients $a_i$ sont des constantes et les variables $x_i$ sont les variables indépendantes.

Ce score correspond au logit de p où p est la probabilité d'existence d'une fibrose cliniquement significative. Cette probabilité p se calcule avec la formule :

$$\texttt{p = exp(a}_0 \texttt{ + a}_1\texttt{x}_1 \texttt{ + a}_2\texttt{x}_2 \texttt{ + …)/(1+exp(a}_0 \texttt{ + a}_1\texttt{x}_1 \texttt{ + a}_2\texttt{x}_2 \texttt{ + …))}$$
$$\texttt{ou p = 1/(1+exp( - a}_0 \texttt{ - a}_1\texttt{x}_1 \texttt{ - a}_2\texttt{x}_2 \texttt{ - …))}$$

où les coefficients $a_i$ et les variables $x_i$ correspondent à ceux de la formule du score. L'existence d'une lésion (par exemple fibrose cliniquement significative) est déterminée par une probabilité p supérieure à 0,5 (sauf spécification différente). Il faut noter que les termes « score » de régression logistique et « score » SNIFF ne correspondent pas au même terme des équations précédentes. En application clinique SNIFF correspond à p.

Nous donnons ci-dessous les tableaux pour chaque score SNIFF avec dans la première colonne le nom de chaque variable indépendante, dans la deuxième colonne la valeur du coefficient $a_i$ associé (appelé β dans le texte ci-dessous et souvent dans la littérature et B dans les tableaux ci-dessous), puis sa déviation standard (appelée E.S dans les tableaux ci-dessous) puis son degré de significativité (appelé signif dans les tableaux ci-dessous) et les 2 dernières colonnes donnent l'intervalle de confiance de exp ($a_i$), c'est-à-dire l'intervalle de confiance (appelé IC dans les tableaux ci-dessous) de l'odds-ratio correspondant (appelé exp(β) dans les tableaux).

Pour chaque score SNIFF, tel que défini dans les variantes de l'invention ci-dessus, la valeur prédictive globale du modèle est reflétée par le « pourcentage global » de sujets bien classés dans un deuxième tableau.

Pour chaque score, dans l'équation applicable, le coefficient $\beta_i$ de chaque variable $x_i$ indépendante peut varier de la valeurs β donné dans le tableau correspondant audit score $\pm$ 3,3 déviations standards, valeur également donnée dans les tableaux. De même, $a_0$ peut varier de la valeur de la constante donnée dans le tableau $\pm$ 3,3 déviations standards.

A titre d'exemple et sur la base des tableaux ci-après, l'homme du métier qui souhaite mettre en oeuvre le score SNIFF 4a à 4 marqueurs utilisera la formule suivante : p = 1/ (1+exp (- $a_0$ - $a_1$ (AH en $\mu$g/l) - $a_2$ (TP en %) - $a_3$ (A2M en mg/dl) - $a_4$(AGE en ans)) avec

- $a_0$ compris entre -3,130 et 7,860 (2,365 $\pm$ 3,3*1,665) et, de préférence, $a_0$ vaut 2,365
- $a_1$ compris entre -0,002 et 0,024 (0,011 $\pm$ 3,3*0,004) et, de préférence, $a_1$ vaut 0,011
- $a_2$ compris entre -0,118 et -0,006 (-0,062 $\pm$ 3, 3*0,017) et, de préférence, $a_2$ vaut -0,062
- $a_3$ compris entre 0,003 et 0,009 (0,006 $\pm$ 3,3*0,001) et, de préférence, $a_3$ vaut 0,006
- $a_4$ compris entre -0,016 et 0,076 (0,030 $\pm$ 3,3*0,014) et, de préférence, $a_4$ vaut 0,030.

Le score SNIFF est exprimé de façon brute (tous les sujets sont inclus) ou optimisée, dans ce cas les sujets extrêmes, caractérisés par un résidu studentisé supérieur à 3, sont écartés de l'analyse. Leur nombre est toujours réduit, en règles ≤ 5%. Pour cette raison, parmi les tableaux fournis ci-après, certains indiqués par un « o » comme par exemple SNIFF 4ao fournissent des coefficients β obtenus après cette optimisation.

De plus, l'homme du métier qui souhaite utiliser des scores dans le cadre de la présente invention pour lesquels les différentes constantes $a_0$ et $a_1$ n'ont pas été fournies dans la présente invention est capable de déterminer lesdites constantes. Il est nécessaire de disposer alors d'une base de données comportant les variables indépendantes utilisées (comme mesurées à l'étape a et b) et une population de sujets ayant la pathologie étudiée (alcool et/ou virus ou stéatose), idéalement plusieurs centaines de sujets, puis de calculer les coefficients $a_1$ (ou $\beta$) comme indiqué à l'étape c et comme expliqué ci-dessus. La variable dépendante est la lésion recherchée, par exemple une fibrose cliniquement significative définie par un score Métavir $\geq 2$.

**1. Pour SNIFF 4a (3 marqueurs de fibrose + age) :**

| Variable | B | E.S. | Signif. | Exp(B) | IC pour Exp(B) 95,0% | |
|---|---|---|---|---|---|---|
| | | | | | Inférieur | Supérieur |
| AH | 0,011 | 0,004 | 0,004 | 1,011 | 1,003 | 1,018 |
| TP | -0,062 | 0,017 | 0 | 0,94 | 0.91 | 0,971 |
| A2M | 0,006 | 0,001 | 0 | 1,006 | 1,003 | 1,009 |
| AGE | 0,03 | 0,014 | 0,028 | 1,03 | 1,003 | 1,058 |
| Constante | 2,365 | 1,665 | 0,156 | 10,641 | | |

**Tableau de classification. La valeur de césure est ,500**

| | Observé | | Prévu | | |
|---|---|---|---|---|---|
| | | | F0+1 vs 2-4 | | |
| | | | ,00 | 1,00 | Pourcentage correct |
| Etape 4 | F0+1 vs 2-4 | ,00 | 107 | 27 | 79,9 |
| | | 1,00 | 37 | 127 | 77,4 |
| | Pourcentage global | | | | **78,5** |

**2. Pour SNIFF 4ao (3 marqueurs de fibrose + age) :**

| | B | E.S. | Signif. | Exp(B) | IC pour Ex)(B) 95,0% | |
|---|---|---|---|---|---|---|
| | | | | | Inférieur | Supérieur |
| AH | ,011 | ,004 | ,007 | 1,011 | 1,003 | 1,020 |
| TP | -,084 | ,019 | ,000 | ,919 | ,886 | ,955 |
| A2M | ,008 | ,002 | ,000 | 1,009 | 1,005 | 1,012 |
| AGE | ,046 | ,015 | ,002 | 1,047 | 1,017 | 1,078 |
| Constante | 3,232 | 1,843 | ,080 | 25,334 | | |

**Tableau de classification. La valeur de césure est ,500**

| | Observé | | Prévu | | |
|---|---|---|---|---|---|
| | | | F0+1 vs 2-4 | | |
| | | | ,00 | 1,00 | Pourcentage correct |
| Etape | F0+1 vs 2-4 | ,00 | 105 | 25 | 80,8 |
| | | 1,00 | 35 | 127 | 78,4 |
| | Pourcentage global | | | | **79,5** |

**3. Pour SNIFF 4b à 3 marqueurs de fibrose + age :**

|  | B | E.S. | Signif. | Exp(B) | IC pour Exp(B) 95,0% | |
|---|---|---|---|---|---|---|
|  |  |  |  |  | Inférieur | Supérieur |
| TP | -,067 | ,016 | ,000 | ,936 | ,906 | ,966 |
| A2M | ,005 | ,002 | ,001 | 1,005 | 1,002 | 1,008 |
| AGE | ,049 | ,013 | ,000 | 1,050 | 1,023 | 1,077 |
| ASAT | ,018 | ,005 | ,000 | 1,019 | 1,009 | 1,028 |
| Constante | 2,024 | 1,647 | ,219 | 7,567 |  |  |

**Tableau de classification(a)**

| Observé | | | Prévu | | |
|---|---|---|---|---|---|
|  |  |  | F0+1 vs 2-4 | |  |
|  |  |  | ,00 | 1,00 | Pourcentage correct |
| Etape 4 | F0+1 vs 2-4 | ,00 | 106 | 29 | 78,5 |
|  |  | 1,00 | 39 | 132 | 77,2 |
|  | Pourcentage global | |  |  | **77,8** |
| a La valeur de césure est ,500 | | | | | |

**4. Pour SNIFF 4bo à 3 marqueurs de fibrose + age :**

|  | B | E.S. | Signif. | Exp(B) | IC pour Exp(B) 95,0% | |
|---|---|---|---|---|---|---|
|  |  |  |  |  | Inférieur | Supérieur |
| TP | -,091 | ,020 | ,000 | ,913 | ,878 | ,949 |
| ASAT | ,023 | ,006 | ,000 | 1,023 | 1,012 | 1,035 |
| A2M | ,008 | ,002 | ,000 | 1,008 | 1,005 | 1,012 |
| AGE | ,072 | ,015 | ,000 | 1,074 | 1,042 | 1,107 |
| Constante | 2,412 | 1,902 | ,205 | 11,154 |  |  |

**Tableau de classification. La valeur de césure est ,500.**

| Observé | | | Prévu | | |
|---|---|---|---|---|---|
|  |  |  | F0+1 vs 2-4 | | Pourcentage correct |
|  |  |  | ,00 | 1,00 |  |
|  | F0+1 vs 2-4 | ,00 | 102 | 26 | 79,7 |
|  |  | 1,00 | 36 | 134 | 78,8 |
|  | Pourcentage global | |  |  | 79,2 |

**5. Pour SNIFF 5 à 4 marqueurs de fibrose + age :**

|  |  | E.S. | Signif. | Exp(B) | IC pour Exp(B) 95,0% | |
|---|---|---|---|---|---|---|
|  |  |  |  |  | Inférieur | Supérieur |
| PLAQUETTES | -,007 | ,002 | ,002 | ,993 | ,988 | ,997 |
| TP | -,059 | ,017 | ,000 | ,943 | ,912 | ,975 |
| ASAT | ,015 | ,005 | ,002 | 1,015 | 1,005 | 1,025 |
| A2M | ,005 | ,002 | ,001 | 1,005 | 1,002 | 1,009 |
| AGE | ,040 | ,013 | ,003 | 1,041 | 1,014 | 1,069 |
| Constante | 3,285 | 1,736 | ,058 | 26,707 |  |  |

### Tableau de classification(a)

|  | Observé |  | Prévu | | |
|---|---|---|---|---|---|
|  |  |  | F0+1 vs 2-4 | |  |
|  |  |  | ,00 | 1,00 | Pourcentage correct |
| Etape 5 | F0+1 vs 2-4 | ,00 | 110 | 23 | 82,7 |
|  |  | 1,00 | 36 | 135 | 78,9 |
|  | Pourcentage global |  |  |  | 80,6 |
| a La valeur de césure est ,500 | | | | | |

**6. Pour SNIFF 50 à 4 marqueurs de fibrose + age :**

|  | B | E.S. | Signif. | Exp(B) | IC pour | Exp(B) 95,0% |
|---|---|---|---|---|---|---|
|  |  |  |  |  | Inférieur | Supérieur |
| TP | -,082 | ,020 | ,000 | ,921 | ,885 | ,959 |
| A2M | ,009 | ,002 | ,000 | 1,009 | 1,005 | 1,013 |
| AGE | ,058 | ,015 | ,000 | 1,059 | 1,028 | 1,092 |
| PLQ | -,008 | ,003 | ,002 | ,992 | ,986 | ,997 |
| ASAT | ,020 | ,006 | ,001 | 1,020 | 1,009 | 1,032 |
| Constante | 4,034 | 2,004 | ,044 | 56,508 |  |  |

### Tableau de classification. La valeur de césure est ,500.

|  | Observé |  | Prévu | |
|---|---|---|---|---|
|  |  |  | F0+1 vs 2-4 | Pourcentage correct |
|  |  |  | ,00 | 1,00 |
| F0+1 vs 2-4 | ,00 | 102 | 25 | 80,3 |
|  | 1,00 | 32 | 138 | 81,2 |
| Pourcentage global |  |  |  | 80,8 |

**7. Pour SNIFF 6 à 5+1 marqueurs de fibrose :**

|  | 8 | E.S. | Signif. | Exp(B) | IC pour Exp(B) 95,0% | |
|---|---|---|---|---|---|---|
|  |  |  |  |  | Inférieur | Supérieur |
| PLAQUETTES | -,008 | ,002 | ,001 | ,992 | ,987 | ,996 |
| ASAT | ,010 | ,005 | ,038 | 1,010 | 1,001 | 1,020 |
| URÉE | -,266 | ,084 | ,002 | ,767 | ,650 | ,904 |
| HYALU | ,023 | ,006 | ,000 | 1,023 | 1,011 | 1,035 |
| AMTRI | ,006 | ,001 | ,000 | 1,006 | 1,003 | 1,009 |
| Constante | ,050 | ,774 | ,948 | 1,052 |  |  |

Avec AMTRI : (A2M / TP) x 100

**Tableau de classification(a)**

|  | Observé | | | Prévu | | |
|---|---|---|---|---|---|---|
|  |  |  |  | F0+1 vs 2-4 | | |
|  |  |  |  | ,00 | 1,00 | Pourcentage correct |
| Etape 5 | F0+1 vs 2-4 | ,00 | | 110 | 22 | 83,3 |
|  |  | 1,00 | | 35 | 130 | 78,8 |
|  | Pourcentage global | | | | | **80,8** |
| a La valeur de césure est ,500 | | | | | | |

**8. Pour SNIFF 6o optimisé à 5+1 marqueurs de fibrose :**

**Variables dans l'équation**

|  |  | B | E.S. | Wald | ddl | Signif. | Exp(B) |
|---|---|---|---|---|---|---|---|
| Etape | PLAQUETTES | -,010 | ,003 | 12,743 | 1 | ,000 | ,990 |
|  | ASAT | ,011 | ,005 | 4,295 | 1 | ,038 | 1,011 |
|  | URÉE | -,365 | ,096 | 14,434 | 1 | ,000 | ,694 |
|  | AH | ,037 | ,009 | 18,482 | 1 | ,000 | 1,038 |
|  | AMTRI | ,007 | ,002 | 21,531 | 1 | ,000 | 1,007 |
|  | Constante | ,171 | ,881 | ,038 | 1 | ,846 | 1.187 |

**Tableau de classification(a)**

|  | Observé | | | Prévu | | |
|---|---|---|---|---|---|---|
|  |  |  |  | F0+1 vs 2-4 | | Pourcentage correct |
|  |  |  |  | ,00 | 1,00 |  |
| Etape | F0+1 vs 2-4 | ,00 | | 106 | 22 | 82,8 |
|  |  | 1,00 | | 30 | 135 | 81,8 |
|  | Pourcentage global | | | | | 82,3 |
| a La valeur de césure est ,500 | | | | | | |

Avec AMTRI : (A2M / TP) x 100

**9. Pour SNIFF 7 à 6 marqueurs de fibrose + age :**

| | B | E.S. | Signif. | Exp(B) | IC pour Exp(B) 95,0% | |
|---|---|---|---|---|---|---|
| | | | | | Inférieur | Supérieur |
| PLAQUETTES | -,007 | ,003 | ,004 | ,993 | ,988 | ,998 |
| ASAT | ,012 | ,005 | ,021 | 1,012 | 1,002 | 1,022 |
| URÉE | -,270 | ,088 | ,002 | ,764 | ,643 | ,907 |
| HYALU | ,021 | ,006 | ,001 | 1,021 | 1,009 | 1,033 |
| TP | -,049 | ,018 | ,007 | ,952 | ,919 | ,987 |
| A2M | ,005 | ,002 | ,003 | 1,005 | 1,002 | 1,008 |
| AGE | ,027 | ,015 | ,063 | 1,028 | ,998 | 1,058 |
| Constante | 3,718 | 1,929 | ,054 | 41,173 | | |

**Tableau de classification(a)**

| | Observé | | Prévu | | |
|---|---|---|---|---|---|
| | | | F0+1 vs 2-4 | | |
| | | | ,00 | 1,00 | Pourcentage correct |
| Etape 7 | F0+1 vs 2-4 | ,00 | 111 | 21 | 84,1 |
| | | 1,00 | 32 | 132 | 80,5 |
| | Pourcentage global | | | | **82,1** |
| a La valeur de césure est ,500 | | | | | |

Variante **SNIFF 7a** avec césure différente pour supprimer les faux négatifs Métavir F3, les coefficients $\beta$ sont inchangés.

| | Observé | | Prévu | | |
|---|---|---|---|---|---|
| | | | F0+1 vs 2-4 | | Pourcentage correct |
| | | | ,00 | 1,00 | |
| | F0+1 vs 2-4 | ,00 | 90 | 42 | 68,2 |
| | | 1,00 | 19 | 145 | 88,4 |
| | Pourcentage global | | | | 79,4 |
| La valeur de césure est ,370 | | | | | |

**10. Pour SNIFF 7o optimisé à 6 marqueurs de fibrose + age :**

| | B | E.S. | Signif. | Exp(B) | IC pour Exp(B) 95,0% | |
|---|---|---|---|---|---|---|
| | | | | | Inférieur | Supérieur |
| PLAQUETTES | -,010 | ,003 | ,001 | ,990 | ,984 | ,996 |
| ASAT | ,014 | ,006 | ,009 | 1,015 | 1,004 | 1,026 |
| URÉE | -,401 | ,105 | ,000 | ,669 | ,544 | ,823 |
| HYALU | ,038 | ,009 | ,000 | 1,039 | 1,020 | 1,058 |
| TP | -,062 | ,021 | ,003 | ,940 | ,902 | ,979 |
| A2M | ,006 | ,002 | ,002 | 1,006 | 1,002 | 1,009 |
| AGE | ,042 | ,017 | ,012 | 1,043 | 1,009 | 1,078 |
| Constante | 4,873 | 2,214 | ,028 | 130,764 | | |

**Tableau de classification(a)**

| | Observé | | Prévu | | |
|---|---|---|---|---|---|
| | | | F0+1 vs 2-4 | | |
| | | | ,00 | 1,00 | Pourcentage correct |
| Etape 7 | F0+1 vs 2-4 | ,00 | 108 | 20 | 84,4 |
| | | 1,00 | 29 | 133 | 82,1 |
| | Pourcentage global | | | | 83,1 |
| a La valeur de césure est ,500 | | | | | |

Variante **SNIFF 7bo** optimisé avec césure différente pour supprimer les faux négatifs Métavir F3, les coefficients β sont inchangés.

| | Observé | | Prévu | | |
|---|---|---|---|---|---|
| | | | F0+1 vs 2-4 | | Pourcentage correct |
| | | | ,00 | 1,00 | |
| | F0+1 vs 2-4 | ,00 | 86 | 42 | 67,2 |
| | | 1,00 | 15 | 147 | 90,7 |
| | Pourcentage global | | | | 80,3 |
| La valeur de césure est ,290 | | | | | |

**11. Pour SNIFFA 3 à 3 marqueurs de fibrose :**

| | B | E.S. | Signif. | Exp(B) | IC pour Exp(B) 95,0% | |
|---|---|---|---|---|---|---|
| | | | | | Inférieur | Supérieur |
| TP | -,161 | ,047 | ,001 | ,851 | ,776 | ,934 |
| ALAT | -,020 | ,009 | ,031 | ,980 | ,963 | ,998 |
| PAL | ,030 | ,011 | ,007 | 1,031 | 1,008 | 1,054 |
| Constante | 13,510 | 4,556 | ,003 | 736506,803 | | |

**Tableau de classification(a)**

| | Observé | | Prévu | | |
|---|---|---|---|---|---|
| | | | F0+1 vs 2-4 | | Pourcentage |
| | | | ,00 | 1,00 | correct |
| | F0+1 vs 2-4 | ,00 | 24 | 5 | 82,8 |
| | | 1,00 | 8 | 57 | 87,7 |
| | Pourcentage global | | | | 86,2 |
| a La valeur de césure est ,500 | | | | | |

**12. Pour SNIFFA 3o à 3 marqueurs de fibrose :**

|  | B | E.S. | Signif. | Exp(B) | IC pour Exp(B) 95,0% | |
|---|---|---|---|---|---|---|
|  |  |  |  |  | Inférieur | Supérieur |
| TP | -,301 | ,095 | ,002 | ,740 | ,614 | ,892 |
| ALAT | -,036 | ,013 | ,007 | ,965 | ,940 | ,990 |
| PAL | ,040 | ,016 | ,010 | 1,041 | 1,010 | 1,073 |
| Constante | 27,447 | 9,265 | ,003 | 831966014903,050 |  |  |

**Tableau de classification(a)**

| Observé | | | Prévu | | |
|---|---|---|---|---|---|
|  |  |  | F0+1 vs 2-4 | | |
|  |  |  | ,00 | 1,00 | Pourcentage correct |
| F0+1 vs 2-4 | | ,00 | 23 | 3 | 88,5 |
|  |  | 1,00 | 4 | 61 | 93,8 |
| Pourcentage global | | | | | 92,3 |
| a La valeur de césure est ,500 | | | | | |

**13. Pour SNIFFA 4a à 3 marqueurs de fibrose + age :**

|  | B | E.S. | Signif. | Exp(B) | IC pour Exp(B) 95,0% | |
|---|---|---|---|---|---|---|
|  |  |  |  |  | Inférieur | Supérieur |
| AGE | -,099 | ,049 | ,042 | ,906 | ,823 | ,996 |
| ALAT | -,032 | ,015 | ,027 | ,968 | ,941 | ,996 |
| HYALU | ,036 | ,013 | ,007 | 1,036 | 1,010 | 1,064 |
| A2M | ,019 | ,008 | ,017 | 1,019 | 1,003 | 1,035 |
| Constante | -,310 | 2,437 | ,899 | ,734 |  |  |

**Tableau de classification(a)**

| Observé | | | | Prévu | | |
|---|---|---|---|---|---|---|
|  |  |  |  | F0+1 vs 2-4 | | |
|  |  |  |  | ,00 | 1,00 | Pourcentage correct |
| Etape 1 | F0+1 vs 2-4 | | ,00 | 23 | 4 | 85,2 |
|  |  |  | 1,00 | 7 | 54 | 88,5 |
|  | Pourcentage global | | | | | 87,5 |
| a La **valeur de césure** est **,500** | | | | | | |

**14. Pour SNIFFA 4ao à 3 marqueurs de fibrose + age:**

**Variables dans l'équation**

|  | B | E.S. | Wald | ddl | Siqnif. | Exp(B) | IC pour Exp(B) 95.0% | |
|---|---|---|---|---|---|---|---|---|
|  |  |  |  |  |  |  | Inférieur | Supérieur |
| ALAT | -,042 | ,017 | 6,092 | 1 | ,014 | ,959 | ,927 | ,991 |
| AH | ,034 | ,012 | 7,694 | 1 | ,006 | 1,034 | 1,010 | 1,059 |
| A2M | ,029 | ,012 | 6,400 | 1 | ,011 | 1,030 | 1,007 | 1,053 |
| AGE | -,176 | ,072 | 5,968 | 1 | ,015 | ,838 | ,728 | ,966 |
| Constante | 1,038 | 2,549 | ,166 | 1 | ,684 | 2,825 |  |  |

**Tableau de classification(a)**

|  | Observé | | Prévu | | |
|---|---|---|---|---|---|
|  |  |  | F0+1 vs 2-4 | | |
|  |  |  | ,00 | 1,00 | Pourcentage correct |
| Etape | F0+1 vs 2-4 | ,00 | 24 | 3 | 88,9 |
|  |  | 1,00 | 4 | 55 | 93,2 |
|  | Pourcentage global | |  |  | 91,9 |
| a La valeur de césure est ,500 | | | | | |

**15. Pour SNIFFA 4b à 4 marqueurs de fibrose :**

|  | B | E.S. | Signif. | Exp(B) | IC pour Exp(B) 95,0% | |
|---|---|---|---|---|---|---|
|  |  |  |  |  | Inférieur | Supérieur |
| TP | -,187 | ,054 | ,001 | ,830 | ,746 | ,923 |
| ALAT | -,026 | ,010 | ,012 | ,974 | ,955 | ,994 |
| PAL | ,036 | ,012 | ,004 | 1,036 | 1,012 | 1,061 |
| RAT | -,739 | ,427 | ,083 | ,477 | ,207 | 1,103 |
| Constante | 16,629 | 5,327 | ,002 | 16674698,481 |  |  |

**Tableau de classification(a)**

|  | Observé | | Prévu | | |
|---|---|---|---|---|---|
|  |  |  | F0+1 vs 2-4 | | |
|  |  |  | ,00 | 1,00 | Pourcentage correct |
|  | F0+1 vs 2-4 | ,00 | 25 | 4 | 86,2 |
|  |  | 1,00 | 7 | 58 | 89,2 |
|  | Pourcentage global | |  |  | 88,3 |
| a La valeur de césure est ,500 | | | | | |

Avec RAT = ASAT / ALAT

**16. Pour SNIFFA 4bo à 4 marqueurs de fibrose :**

|  | B | E.S. | Signif. | Exp(B) | IC pour Exp(B) 95,0% | |
|---|---|---|---|---|---|---|
|  |  |  |  |  | Inférieur | Supérieur |
| TP | -,435 | ,165 | ,008 | ,648 | ,469 | ,894 |
| ALAT | -,058 | ,023 | ,012 | ,944 | ,902 | ,988 |
| PAL | ,088 | ,033 | ,007 | 1,092 | 1,025 | 1,164 |
| RAT | -1,958 | ,818 | ,017 | ,141 | ,028 | ,701 |
| Constante | 39,515 | 15,768 | ,012 | 144962082235443400,000 | | |

**Tableau de classification(a)**

| Observé | | | Prévu | | |
|---|---|---|---|---|---|
|  |  | F0+1 vs 2-4 | | |
|  |  | ,00 | 1,00 | Pourcentage correct |
| F0+1 vs 2-4 | ,00 | 23 | 2 | 92,0 |
|  | 1,00 | 4 | 59 | 93,7 |
| Pourcentage global | | | | 93,2 |
| a La valeur de césure est ,500 | | | | |

Avec RAT = ASAT / ALAT

Variante **SNIFF 4b2o** optimisé avec césure différente pour supprimer les faux positifs Métavir F0, les coefficients β sont inchangés.

| Observé | | | Prévu | |
|---|---|---|---|---|
|  |  | F0+1 vs 2-4 | | Pourcentage correct |
|  |  | ,00 | 1,00 |  |
| F0+1 vs 2-4 | ,00 | 24 | 1 | 96,0 |
|  | 1,00 | 4 | 59 | 93,7 |
| Pourcentage global | | | | 94,3 |
| La valeur de césure est ,550 | | | | |

**17. Pour SNIFFA 4c à 3 marqueurs de fibrose + age :**

|  | B | E.S. | Signif. | Exp(B) | IC pour Exp(B) 95,0% | |
|---|---|---|---|---|---|---|
|  |  |  |  |  | Inférieur | Supérieur |
| AH | ,032 | ,012 | ,007 | 1,032 | 1,009 | 1,056 |
| A2M | ,015 | ,008 | ,068 | 1,015 | ,999 | 1,032 |
| AGE | -,140 | ,058 | ,015 | ,869 | ,776 | ,973 |
| TP | -,169 | ,067 | ,012 | ,845 | ,741 | ,963 |
| Constante | 16,541 | 7,858 | ,035 | 15263638,220 | | |

Tableau de classification. La césure est à 0,50.

| Observé | | | Prévu | | |
|---|---|---|---|---|---|
| | | | F0+1 vs 2-4 | | Pourcentage correct |
| | | | ,00 | 1,00 | |
| F0+1 vs 2-4 | | ,00 | 25 | 2 | 92,6 |
| | | 1,00 | 5 | 56 | 91,8 |
| Pourcentage global | | | | | 92,0 |

**18. Pour SNIFFA 4co à 3 marqueurs de fibrose + age :**

| | B | E.S. | Signif. | Exp(B) | IC pour Exp(B) 95,0% | |
|---|---|---|---|---|---|---|
| | | | | | Inférieur | Supérieur |
| AH | ,078 | ,031 | ,013 | 1,081 | 1,017 | 1,150 |
| A2M | ,049 | ,024 | ,047 | 1,050 | 1,001 | 1,101 |
| AGE | -,560 | ,219 | ,011 | ,571 | ,372 | ,878 |
| TP | -,629 | ,266 | ,018 | ,533 | ,316 | ,898 |
| Constante | 68,252 | 29,471 | ,021 | 438086735113701800000000000000,0 | | |

Tableau de classification. La césure est à 0,62.

| Observé | | | Prévu | | |
|---|---|---|---|---|---|
| | | | F0+1 vs 2-4 | | Pourcentage correct |
| | | | ,00 | 1,00 | |
| F0+1 vs 2-4 | | ,00 | 25 | 0 | 100,0 |
| | | 1,00 | 2 | 58 | 96,7 |
| Pourcentage global | | | | | 97,6 |

**19. Pour SNIDAFF 6a à 5 marqueurs de fibrose :**

| | B | E.S. | Signif. | Exp(B) | IC pour Exp(B) 95,0% | |
|---|---|---|---|---|---|---|
| | | | | | Inférieur | Supérieur |
| AGE | ,031 | ,012 | ,008 | 1,032 | 1,008 | 1,056 |
| PLAQUETTES | -,006 | ,002 | ,002 | ,994 | ,990 | ,998 |
| TP | -,076 | ,015 | ,000 | ,927 | ,900 | ,956 |
| ASAT | ,008 | ,004 | ,040 | 1,008 | 1,000 | 1,016 |
| PAL | ,007 | ,003 | ,036 | 1,007 | 1,000 | 1,014 |
| A2M | ,006 | ,001 | ,000 | 1,006 | 1,003 | 1,009 |
| Constante | 4,575 | 1,602 | ,004 | 97,048 | | |

**Tableau de classification(a) "**

| Observé | | | Prévu | | |
|---|---|---|---|---|---|
| | | | F0+1 vs 2-4 | | |
| | | | ,00 | 1,00 | Pourcentage correct |
| F0+1 vs 2-4 | ,00 | | 114 | 40 | 74,0 |
| | 1,00 | | 34 | 189 | 84,8 |
| Pourcentage global | | | | | 80,4 |
| a La valeur de césure est ,470 | | | | | |

**20. Pour SNIDAFF 6b à 5 marqueurs de fibrose :**

| | B | E.S. | Signif. | Exp(B) | IC pour Exp(B) 95,0% | |
|---|---|---|---|---|---|---|
| | | | | | Inférieur | Supérieur |
| AGE | ,061 | ,012 | ,000 | 1,063 | 1,038 | 1,089 |
| PLAQUETTES | -,010 | ,002 | ,000 | ,990 | ,986 | ,995 |
| TP | -,101 | ,017 | ,000 | ,904 | ,874 | ,935 |
| ASAT | ,017 | ,004 | ,000 | 1,017 | 1,008 | 1,026 |
| PAL | ,015 | ,004 | ,000 | 1,015 | 1,007 | 1,023 |
| URÉE | -,157 | ,066 | ,017 | ,855 | ,751 | ,973 |
| Constante | 7,817 | 1,741 | ,000 | 2483,002 | | |

**Tableau de classification(a)**

| Observé | | | Prévu | | |
|---|---|---|---|---|---|
| | | | F0+1 vs 2-4 | | |
| | | | ,00 | 1,00 | Pourcentage correct |
| F0+1 vs 2-4 | ,00 | | 109 | 60 | 64,5 |
| | 1,00 | | 35 | 215 | 86,0 |
| Pourcentage global | | | | | 77,3 |
| a La valeur de césure est ,400 | | | | | |

**21. Pour SNIAH :**

**Variables dans l'équation**

| | | B | E.S. | Wald | ddl | Signif. | Exp(B) |
|---|---|---|---|---|---|---|---|
| Etape | ALAT | ,010 | ,002 | 22,575 | 1 | ,000 | 1,010 |
| | Constante | -,474 | ,200 | 5,601 | 1 | ,018 | ,622 |

**Tableau de classification(a)**

| | Observé | | | Prévu | | |
|---|---|---|---|---|---|---|
| | | | | ACTIVICS | | |
| | | | | ,00 | 1,00 | Pourcentage correct |
| Etape | ACTIVICS | ,00 | | 57 | 93 | 38,0 |
| | | 1,00 | | 33 | 193 | 85,4 |
| | Pourcentage global | | | | | 66,5 |
| a La valeur de césure est ,500 | | | | | | |

**22. Pour SNIAH o :**

| | B | E.S. | Signif. | Exp(B) | IC pour Exp(B) 95,0% | |
|---|---|---|---|---|---|---|
| | | | | | Inférieur | Supérieur |
| ALAT | ,018 | ,003 | ,000 | 1,018 | 1,012 | 1,024 |
| Constante | -1,003 | ,237 | ,000 | ,367 | | |

| | Observé | | | Prévu | | |
|---|---|---|---|---|---|---|
| | | | | ACTIVICS | | Pourcentage correct |
| | | | | ,00 | 1,00 | |
| | ACTIVICS | ,00 | | 73 | 71 | 50,7 |
| | | 1,00 | | 59 | 167 | 73,9 |
| | Pourcentage global | | | | | 64,9 |
| a La **valeur** de césure est ,500 | | | | | | |

**23. Pour SNIFFSA 3 :**

**Variables dans l'équation**

| | B | E.S. | Signif. | Exp(B) | IC pour Exp(B) 95,0% | |
|---|---|---|---|---|---|---|
| | | | | | Inférieur | Supérieur |
| PLAQUETTES | -,012 | ,006 | ,047 | ,988 | ,976 | 1,000 |
| TP | -,090 | ,040 | ,025 | ,914 | ,844 | ,989 |
| NA | -,348 | ,158 | ,027 | ,706 | ,518 | ,962 |
| Constante | 59,293 | 22,781 | ,009 | 56312993793815500000000000,000 | | |

**Tableau de classification(a)**

| | Observé | | | Prévu | | |
|---|---|---|---|---|---|---|
| | | | | F<2 vs >=2 | | |
| | | | | ,00 | 1,00 | Pourcentage correct |
| | F<2 vs >=2 | ,00 | | 18 | 2 | 90,0 |

(suite)

| | Observé | | Prévu | | |
|---|---|---|---|---|---|
| | | | F<2 vs >=2 | | |
| | | | ,00 | 1,00 | Pourcentage correct |
| | 1,00 | | 1 | 20 | 95,2 |
| | Pourcentage global | | | | 92,7 |
| a La valeur de césure est ,500 | | | | | |

**24. Pour SNIFFSA 4a :**

| | B | E.S. | Signif. | Exp(B) | IC pour Exp(B) 95,0% | |
|---|---|---|---|---|---|---|
| | | | | | Inférieur | Supérieur |
| TP | -,143 | ,051 | ,005 | ,866 | ,783 | ,958 |
| AGE | ,130 | ,049 | ,008 | 1,139 | 1,034 | 1,254 |
| GLYCEMIE | ,566 | ,333 | ,089 | 1,761 | ,917 | 3,383 |
| ASAT | ,025 | ,014 | ,073 | 1,025 | ,998 | 1,053 |
| Constante | 1,134 | 5,286 | ,830 | 3,107 | | |

| | Observé | | Prévu | | |
|---|---|---|---|---|---|
| | | | F<2 vs >=2 | | Pourcentage correct |
| | | | ,00 | 1,00 | |
| F<2 vs >=2 | ,00 | | 25 | 1 | 96,2 |
| | 1,00 | | 4 | 25 | 86,2 |
| | Pourcentage global | | | | 90,9 |
| **La valeur de césure est ,500** | | | | | |

**25. Pour SNIFFSA 4ao :**

| | B | E.S. | Signif. | Exp(B) | IC pour Exp(B) 95.0% | |
|---|---|---|---|---|---|---|
| | | | | | Inférieur | Supérieur |
| TP | -,362 | ,184 | ,050 | ,696 | ,485 | ,999 |
| AGE | ,407 | ,205 | ,047 | 1,503 | 1,006 | 2,245 |
| GLYCEMIE | 1,424 | ,962 | ,139 | 4,154 | ,630 | 27,384 |
| ASAT | ,089 | ,053 | ,092 | 1,093 | ,986 | 1,212 |
| Constante | -2,362 | 8,803 | ,788 | ,094 | | |

| | Observé | | Prévu | | |
|---|---|---|---|---|---|
| | | | F<2 vs >=2 | | Pourcentage correct |
| | | | ,00 | 1,00 | |
| F<2 vs >=2 | ,00 | | 24 | 1 | 96,0 |

(suite)

| Observé | | Prévu | |
|---|---|---|---|
| | | F<2 vs >=2 | Pourcentage correct |
| | | ,00 | 1,00 | |
| 1,00 | 1 | 27 | 96,4 |
| Pourcentage global | | | 96,2 |
| a La valeur de césure est ,500 | | | |

**26. Pour SNIFFSA 4b** :

| | B | E.S. | Signif. | Exp(B) | IC pour Exp(B) 95,0% | |
|---|---|---|---|---|---|---|
| | | | | | Inférieur | Supérieur |
| TP | -,105 | ,047 | ,026 | ,900 | ,821 | ,987 |
| AGE | ,140 | ,057 | ,014 | 1,150 | 1,029 | 1,286 |
| GLYCEMIE | ,931 | ,357 | ,009 | 2,537 | 1,261 | 5,107 |
| TRIGLYCERIDES | -1,889 | 1,023 | ,065 | ,151 | ,020 | 1,122 |
| Constante | -1,697 | 5,243 | ,746 | ,183 | | |

| Observé | | | Prévu | | |
|---|---|---|---|---|---|
| | | | F<2 vs >=2 | | |
| | | | ,00 | 1,00 | Pourcentage correct |
| Etape 1 | F<2 vs >=2 | ,00 | 21 | 2 | 91,3 |
| | | 1,00 | 2 | 22 | 91,7 |
| | Pourcentage global | | | | 91,5 |
| a La valeur de césure est ,500 | | | | | |

**27. Pour SNIFFAV 5:**

**Variables dans l'équation**

| | B | E.S. | Signif. | Exp(B) | IC pour Exp(B) 95,0% | |
|---|---|---|---|---|---|---|
| | | | | | Inférieur | Supérieur |
| PLAQUETTES | -,008 | ,002 | ,000 | ,992 | ,988 | ,997 |
| TP | -,051 | ,017 | ,002 | ,950 | ,920 | ,982 |
| AH | ,019 | ,004 | ,000 | 1,020 | 1,011 | 1,028 |
| A2M | ,007 | ,001 | ,000 | 1,007 | 1,004 | 1,010 |
| URÉE | -,199 | ,065 | ,002 | ,819 | ,721 | ,931 |
| Constante | 4,648 | 1,665 | ,005 | 104,330 | | |

**Tableau de classification(a)**

| | Observé | | | Prévu | | |
|---|---|---|---|---|---|---|
| | | | | F0+1 vs 2-4 | | Pourcentage correct |
| | | | | ,00 | 1,00 | |
| Etape | F0+1 vs 2-4 | ,00 | | 131 | 28 | 82,4 |
| | | 1,00 | | 40 | 186 | 82,3 |
| | Pourcentage global | | | | | 82,3 |
| a La valeur de césure est ,500 | | | | | | |

**28. Pour SNIFFAV 5o :**

| | B | E.S. | Signif. | Exp(B) | IC pour Exp(B) 95,0% | |
|---|---|---|---|---|---|---|
| | | | | | Inférieur | Supérieur |
| PLQ | -,009 | ,002 | ,000 | ,991 | ,986 | ,996 |
| TP | -,076 | ,020 | ,000 | ,927 | ,891 | ,964 |
| URÉE | -,314 | ,083 | ,000 | ,731 | ,621 | ,861 |
| AH | ,035 | ,007 | ,000 | 1,036 | 1,021 | 1,051 |
| A2M | ,008 | ,002 | ,000 | 1,008 | 1,005 | 1,012 |
| Constante | 7,105 | 2,036 | ,000 | 1218,006 | | |

**Tableau de classification**

| | Observé | | | Prévu | | |
|---|---|---|---|---|---|---|
| | | | | F0+1 vs 2-4 | | Pourcentage correct |
| | | | | ,00 | 1,00 | |
| | F0+1 vs 2-4 | ,00 | | 121 | 29 | 80,7 |
| | | 1,00 | | 30 | 194 | 86,6 |
| | Pourcentage global | | | | | **84,2** |
| La valeur de césure est ,490 | | | | | | |

**29. Pour SNIFFAV 6 :**

**Variables dans l'équation**

| | B | E.S. | Signif. | Exp(B) | IC pour Exp(B) 95,0% | |
|---|---|---|---|---|---|---|
| | | | | | Inférieur | Supérieur |
| PLAQUETTES | -,008 | ,002 | ,000 | ,992 | ,988 | ,996 |
| TP | -,052 | ,016 | ,002 | ,950 | ,920 | ,981 |
| AH | ,023 | ,005 | ,000 | 1,024 | 1,014 | 1,033 |
| A2M | ,007 | ,001 | ,000 | 1,007 | 1,004 | 1,010 |
| CAUSE | 1,086 | ,442 | ,014 | 2,963 | 1,247 | 7,043 |
| URÉE | -,271 | ,073 | ,000 | ,762 | ,660 | ,880 |
| Constante | 3,124 | 1,752 | ,075 | 22,737 | | |

**Tableau de classification(a)**

| | Observé | | | Prévu | |
|---|---|---|---|---|---|
| | | | F0+1 vs 2-4 | | Pourcentage correct |
| | | | ,00 | 1,00 | |
| Etape | F0+1 vs 2-4 | ,00 | 133 | 26 | 83,6 |
| | | 1,00 | 38 | 188 | 83,2 |
| | Pourcentage global | | | | 83,4 |
| a La valeur de césure est ,500 | | | | | |

**30. Pour SNIFFAV 6o :**

| | B | E.S. | Signif. | Exp(B) | IC pour Exp(B) 95,0% | |
|---|---|---|---|---|---|---|
| | | | | | Inférieur | Supérieur |
| PLQ | -,010 | ,003 | ,000 | ,990 | ,985 | ,995 |
| TP | -,055 | ,018 | ,002 | ,946 | ,913 | ,981 |
| URÉE | -.396 | ,090 | ,000 | ,673 | ,564 | ,803 |
| AH | ,041 | ,008 | ,000 | 1,042 | 1,026 | 1,058 |
| A2M | ,008 | ,002 | ,000 | 1,008 | 1,005 | 1,011 |
| ETIO | -1,648 | ,517 | ,001 | ,192 | ,070 | ,530 |
| Constante | 5,974 | 1,925 | ,002 | 392,931 | | |

| | Observé | | | Prévu | |
|---|---|---|---|---|---|
| | | | F0+1 vs 2-4 | | Pourcentage correct |
| | | | ,00 | 1,00 | |
| | F0+1 vs 2-4 | ,00 | 123 | 29 | 80,9 |
| | | 1,00 | 36 | 189 | 84,0 |
| | Pourcentage global | | | | 82,8 |

La valeur de césure est ,500. Comme on peut le constater le gain de performance ne se fait pas sur la performance diagnostique (82,8 vs 83,4 %) mais sur d'autres indices de performance comme l'aire sous courbe ROC (0,910 vs 0,890).

Les scores **SNIAFF, SNIAFFA, SNIAFFSA, SNIAFFAV et SNIDIFFAV** ont été obtenus par la méthode statistique appelée régression linéaire multiple avec la procédure suivante :

Dans un premier temps, les variables ont été testées en analyse univariée.

Dans un deuxième temps, les variables significatives en analyse univariée ont été testées en analyse multivariée par régression linéaire avec sélection pas à pas ascendante.

Le modèle statistique linéaire est décrit par l'équation suivante :

$$y_i = a + \beta_1 x_1 + \beta_2 x_2 + \dots$$

où a est la constante, $\beta_i$ est le coefficient de chaque variable $x_i$ indépendante, et $y_i$ est la variable dépendante (aire de fibrose).

Nous donnons ci-dessous les tableaux pour chaque score avec dans la première colonne le nom de chaque variable indépendante, dans la deuxième colonne la valeur du coefficient β puis sa déviation standard, puis le coefficient β standardisé et dans les deux dernières colonnes l'intervalle de confiance à 95 % du coefficient β.

Pour chaque score, tel que défini dans les variantes de l'invention ci-dessus, la valeur prédictive globale du modèle est reflétée, dans un deuxième tableau, par le coefficient R-deux ajusté de chaque modèle qui est le pourcentage de variabilité de $y_i$ expliqué par les variables indépendantes du modèle.

Pour chaque score, dans l'équation applicable, le coefficient $\beta_i$ de chaque variable $x_i$ indépendante peut varier de la valeur β donné dans le tableau correspondant audit score $\pm$ 3,3 déviations standards, valeur également donnée dans le tableau. De même, $a_o$ peut varier de la valeur de la constante donnée dans le tableau $\pm$ 3,3 déviations standards.

**31. Pour SNIAFF 5 à 5 marqueurs de fibrose :**

### Récapitulatif du modèle

| Modèle | R | R-deux | R-deux ajusté | Erreur standard de l'estimation |
|---|---|---|---|---|
| | ,809 | ,655 | ,645 | 3,03260 |
| Avec GAPRI = ((GGT/45) / PLQ) x 100 | | | | |

**32. Pour SNIAFF 6a :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 9,491 | 1,168 | | ,000 | 7,186 | 11, 797 |
| GAPRI | 3,037 | ,317 | 1,033 | ,000 | 2,411 | 3,664 |
| GGT | -,034 | ,005 | -,652 | ,000 | -,044 | -,024 |
| AH | ,015 | ,003 | ,283 | ,000 | ,010 | ,021 |
| APOA1 | -1,666 | ,639 | -,122 | ,010 | -2,927 | -,404 |
| BILI | ,091 | ,037 | ,122 | ,015 | ,018 | ,164 |

### Récapitulatif du modèle

| Modèle | R | R-deux | R-deux ajusté | Erreur standard de l'estimation |
|---|---|---|---|---|
| | ,798 | ,637 | ,625 | 3,13055 |

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 6,739 | ,885 | | ,000 | 4,992 | 8,485 |
| AH | ,017 | ,003 | ,297 | ,000 | ,011 | ,022 |
| GAPRI | 2,945 | ,327 | 1,130 | ,000 | 2,301 | 3,590 |
| GGT | -,037 | ,005 | -,842 | ,000 | -,047 | -,027 |
| BILI | ,106 | ,037 | ,139 | ,005 | ,033 | ,180 |
| A2M | ,005 | ,002 | ,116 | ,020 | ,001 | ,010 |

(suite)

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| URÉE | -,203 | ,089 | -,107 | ,024 | -,378 | -,027 |
| Avec GAPRI = ((GGT/45) / PLQ) x 100 | | | | | | |

**33. Pour SNIAFF 6b :**

**Coefficients**

| Modèle | R | R-deux | R-deux ajusté | Erreur standard de l'estimation |
|---|---|---|---|---|
| | ,802 | ,643 | ,631 | 3,10748 |

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 6,014 | ,981 | | ,000 | 4,077 | 7,950 |
| AH | ,016 | ,003 | ,286 | ,000 | ,010 | ,022 |
| GAPRI | 2,844 | ,327 | 1,094 | ,000 | 2,199 | 3,489 |
| GGT | -,035 | ,005 | -,803 | ,000 | -,045 | -,025 |
| BILI | ,111 | ,037 | ,145 | ,003 | ,038 | ,185 |
| GGLOB | ,156 | ,053 | ,145 | ,004 | ,051 | ,261 |
| URÉE | -,188 | ,088 | -,100 | ,033 | -,362 | -,015 |
| Avec GAPRI = ((GGT/45) / PLQ) x 100 | | | | | | |

**34. Pour SNIAFFA 2 :**

**Récapitulatif du modèle**

| R | R-deux | R-deux ajusté | Erreur standard de l'estimation |
|---|---|---|---|
| ,897 | ,804 | ,798 | 6,15243 |

**Coefficients**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 3,105 | 2,270 | | ,176 | -1,420 | 7,631 |
| A2M | ,019 | ,008 | ,130 | ,019 | ,003 | ,035 |
| AH | ,065 | ,004 | ,854 | ,000 | ,056 | ,073 |

**35. Pour SNIAFFA 3 :**

**Récapitulatif du modèle**

| R | R-deux | R-deux ajusté | Erreur standard de l'estimation |
|---|---|---|---|
| ,902 | ,814 | ,806 | 6,03664 |

**Coefficients**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| AH | ,062 | ,004 | ,824 | ,000 | ,054 | ,071 |
| A2M | ,020 | ,008 | ,134 | ,014 | ,004 | ,035 |
| POIDS | ,124 | ,057 | ,116 | ,032 | ,011 | ,238 |

**36. Pour SNIAFFA 4 à 3 marqueurs de fibrose :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | -17,492 | 5,040 | | ,001 | -27,554 | -7,429 |
| HYAMPRI | 4,242 | ,504 | ,605 | ,000 | 3,235 | 5,249 |
| POIDS | ,255 | ,068 | ,236 | ,000 | ,118 | ,391 |
| PIIIP | 4,010 | 1,273 | ,224 | ,002 | 1,469 | 6,552 |
| A2M | ,024 | ,010 | ,164 | ,013 | ,005 | ,043 |
| avec HYAMPRI : (AH * A2M) / (A2M * 100) | | | | | | |

| Modèle | R | R-deux | R-deux ajusté | Erreur standard de l'estimation |
|---|---|---|---|---|
| | ,866 | ,750 | ,735 | 7,11277 |

**37. Pour SNIAFFA 4o à 3 marqueurs de fibrose :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | -6,880 | 4, 356 | | ,119 | -15,590 | 1,831 |
| HYAMPRI | 5,470 | ,478 | ,752 | ,000 | 4,515 | 6,426 |
| POIDS | ,128 | , 057 | ,119 | ,000 ,028 | ,014 | ,242 |
| A2M | ,016 | , 008 | ,113 | ,034 | ,001 | ,032 |

(suite)

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| PIIIP | 2,521 | 1,064 | ,148 | ,021 | ,394 | 4,649 |

| Modèle | R | R-deux | R-deux ajusté | Erreur standard de l'estimation |
|---|---|---|---|---|
| | ,920 | ,846 | ,836 | 5,49377 |

**38. Pour SNIAFFA 4a à 3 marqueurs de fibrose :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | -10,122 | 4,720 | | ,035 | -19,533 | -,711 |
| HYAMPRI | 4,285 | ,473 | ,624 | ,000 | 3,341 | 5,228 |
| AMTRI | ,022 | ,005 | ,285 | ,000 | ,011 | ,033 |
| POIDS | ,209 | ,071 | ,191 | ,004 | ,067 | ,351 |
| avec HYAMPRI : (AH * A2M) / (A2M * 100), AMTRI : (A2M/ TP) * 100 | | | | | | |

| Modèle | R | R-deux | R-deux ajusté | Erreur standard de l'estimation |
|---|---|---|---|---|
| | ,848 | ,719 | ,707 | 7,49622 |

**39. Pour SNIAFFA 4b à 3 marqueurs de fibrose :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | -10,670 | 4,637 | | ,024 | -19,917 | -1,422 |
| AH | ,042 | ,005 | ,613 | ,000 | ,032 | ,051 |
| POIDS | ,213 | ,070 | ,197 | ,003 | ,073 | ,352 |
| AMTRI | ,023 | .005 | ,300 | ,000 | ,012 | ,033 |
| avec AMTRI : (A2M/ TP) *100 | | | | | | |

| Modèle | R | R-deux | R-deux ajusté | Erreur standard de l'estimation |
|---|---|---|---|---|
| | ,853 | ,727 | ,715 | 7,35058 |

**40. Pour SNIAFFA 4co à 3 marqueurs de fibrose :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 3,693 | 1,680 | | ,032 | ,337 | 7,049 |
| HAMPRI | -,700 | ,314 | -,308 | ,029 | -1,328 | -,073 |
| HYATRI | -,021 | ,007 | -,551 | ,003 | -,035 | -,007 |
| AMPRI | ,026 | ,010 | ,227 | ,009 | ,007 | ,045 |
| HYAMTRI | ,517 | ,158 | ,398 | ,002 | ,201 | ,832 |
| HYAMPRI | 8,853 | 1,293 | 1,243 | ,000 | 6,269 | 11,437 |

avec HAMPRI = (AH * A2M) / (PLQ * 100), HYATRI : (AH / TP) * 100, AMPRI : (A2M / PLQ) * 100, HYAMTRI : (AH * A2M) / (TP * 100), HYAMPRI : (AH * A2M) / (A2M * 100).

| Modèle | R | R-deux | R-deux ajusté | Erreur standard de l'estimation |
|---|---|---|---|---|
| | ,922 | ,849 | ,837 | 5,50329 |

**41. Pour SNIAFFAV 4 :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 20,659 | 4,496 | | ,000 | 11,805 | 29,513 |
| AH | ,026 | ,003 | ,413 | ,000 | ,020 | ,033 |
| TP | -,180 | ,041 | -,236 | ,000 | -,261 | -,098 |
| BILI | ,208 | ,043 | ,238 | ,000 | ,123 | ,292 |
| A2M | ,010 | ,004 | ,110 | ,008 | ,003 | ,017 |

| Modèle | R | R-deux | R-deux ajusté | Erreur standard de l'estimation |
|---|---|---|---|---|
| | ,774 | ,599 | ,593 | 6,30666 |

**42. Pour SNIAFFAV 5 :**

**Récapitulatif du modèle**

| Modèle | R | R-deux | R-deux ajusté | Erreur standard de l'estimation |
|---|---|---|---|---|
| | ,880 | ,775 | ,770 | 3,95555 |

**Coefficients**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 19,405 | 2,884 | | ,000 | 13,724 | 25,085 |
| PLAQUETTES | -,006 | ,003 | -,059 | ,077 | -,013 | ,001 |
| TP | -,063 | ,028 | -,094 | ,025 | -,118 | -,008 |
| URÉE | -,231 | ,105 | -,073 | ,028 | -,437 | -,025 |
| AH | ,049 | ,003 | ,729 | ,000 | ,043 | ,055 |
| Cause | -2,206 | ,667 | -,119 | ,001 | -3,520 | -,893 |

**43. Pour SNIAFFAV 5b :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 21,371 | 4,489 | | ,000 | 12,530 | 30,212 |
| AH | ,026 | ,003 | ,412 | ,000 | ,020 | ,033 |
| TP | -,173 | ,041 | -,227 | ,000 | -,255 | -,092 |
| URÉE | -,294 | ,155 | -,077 | ,060 | -,600 | ,012 |
| BILI | ,197 | ,043 | ,226 | ,000 | ,112 | ,282 |
| A2M | ,011 | ,004 | ,120 | ,004 | ,003 | ,018 |

| Modèle | R | R-deux | R-deux ajusté | Erreur standard de l'estimation |
|---|---|---|---|---|
| | ,778 | ,605 | ,597 | 6,27506 |

**44. Pour SNIAFFAV 5bo :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 11,229 | 3,549 | | ,002 | 4,238 | 18,220 |
| AH | ,037 | ,003 | ,602 | ,000 | ,032 | ,043 |
| TP | -,065 | ,033 | -,095 | ,049 | -,130 | ,000 |
| URÉE | -,264 | ,118 | -,078 | ,027 | -,497 | -,031 |
| BILI | ,174 | ,034 | ,223 | ,000 | ,107 | ,240 |
| A2M | ,007 | ,003 | ,086 | ,015 | ,001 | ,013 |

| Modèle | R | R-deux | R-deux ajusté | Erreur standard de l'estimation |
|---|---|---|---|---|
| | ,848 | ,719 | ,713 | 4,73071 |

**45. Pour SNIAFFAV 5co :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 9,300 | 1,387 | | ,000 | 6,568 | 12,032 |
| AH | ,032 | ,004 | ,496 | ,000 | ,024 | ,041 |
| BILI | ,126 | ,031 | ,164 | ,000 | ,065 | ,187 |
| HYAMTRI | ,313 | ,073 | ,255 | ,000 | ,169 | ,457 |
| etio | -1,972 | ,658 | -,104 | ,003 | -3,268 | -,676 |
| avec HYAMTRI : (AH * A2M) / (TP * 100) | | | | | | |

| Modèle | R | R-deux | R-deux ajusté | Erreur standard de l'estimation |
|---|---|---|---|---|
| | ,888 | ,789 | ,785 | 3,90275 |

**46. Pour SNIAFFAV 8 :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 1,443 | 1,211 | | ,234 | -,941 | 3,828 |
| BILI | ,166 | ,039 | ,192 | ,000 | ,089 | ,243 |
| AMTRI | ,035 | ,007 | ,531 | ,000 | ,020 | ,049 |
| GLOPRI | ,493 | ,078 | ,352 | ,000 | ,339 | ,646 |
| HYAPRI | -,040 | ,006 | -,653 | ,000 | -,053 | -,028 |
| AH | ,050 | ,005 | ,801 | ,000 | ,039 | ,061 |
| A2M | -,029 | ,009 | -,333 | ,002 | -,047 | -,011 |
| GAPRI | ,704 | ,138 | ,321 | ,000 | ,432 | ,976 |
| APRI | -2,120 | ,575 | -,231 | ,000 | -3,252 | -,989 |
| avec AMTRI . (A2M/ TP) * 100, GLOPRI : (GLB/ PLQ) * 100, HYAPRI : (AH / PLQ) * 100, GAPRI = ((GGT/45) / PLQ) * 100, APRI = (ASAT / PLQ) * 100 | | | | | | |

| Modèle | R | R-deux | R-deux ajusté | Erreur standard de l'estimation |
|---|---|---|---|---|
| | ,836 | ,699 | ,689 | 5,45747 |

**47. Pour SNIDIFFAV 4a :**

**Récapitulatif du modèle**

| Modèle | R | R-deux | R-deux ajusté | Erreur standard de l'estimation |
|---|---|---|---|---|
|  | ,826 | ,682 | ,660 | , 11630 |

**Coefficients**

|  | Coefficients non standardisés | | Coefficients standardisés | Signification | Intervalle de confiance à 95% de B | |
|---|---|---|---|---|---|---|
|  | B | Erreur standard | Bêta |  | Borne inférieure | Borne supérieure |
| (constante) | 1,621 | ,169 |  | ,000 | 1,283 | 1,959 |
| TP | -,003 | ,002 | -,248 | ,057 | -,006 | ,000 |
| AH | ,000 | ,000 | ,259 | ,041 | ,000 | ,001 |
| A2M | ,001 | ,000 | ,277 | ,001 | ,000 | ,001 |
| ALB | -,011 | ,003 | -,361 | ,001 | -,017 | -.004 |

**48. Pour SNIDIFFAV 4b**

**Coefficients**

|  | Coefficients non standardisés | | Coefficients standardisés | Signification | Intervalle de confiance à 95% de B | |
|---|---|---|---|---|---|---|
|  | B | Erreur standard | Bêta |  | Borne inférieure | Borne supérieure |
| (constante) | 1,727 | ,136 |  | ,000 | 1,455 | 1,999 |
| AGE | ,002 | ,001 | ,155 | ,061 | ,000 | ,005 |
| ALB | -,012 | ,003 | -,397 | ,000 | -,018 | -,006 |
| A2M | ,001 | ,000 | ,266 | ,001 | ,000 | ,001 |
| TP | -,004 | ,001 | -,372 | ,001 | -,007 | -,002 |

**Récapitulatif du modèle**

| Modèle | R | R-deux | R-deux ajusté | Erreur standard de l'estimation |
|---|---|---|---|---|
|  | ,827 | ,684 | ,662 | ,11631 |

**49. Pour SNIDIFFAV 6 :**

|  | Coefficients non standardisés | | Coefficients standardisés | Signification | Intervalle de confiance à 95% de B | |
|---|---|---|---|---|---|---|
|  | B | Erreur standard | Bêta |  | Borne inférieure | Borne supérieure |
| (constante) | 1,553 | ,159 |  | ,000 | 1,235 | 1,870 |
| AGE | ,003 | ,001 | ,176 | ,031 | ,000 | ,005 |
| ALB | -,010 | ,003 | -,336 | ,002 | -,016 | -,004 |
| A2M | ,001 | ,000 | ,267 | ,001 | ,000 | ,001 |
| TP | -,004 | ,001 | -,338 | ,002 | -,007 | -,002 |

(suite)

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| RAT | ,041 | ,020 | ,167 | ,050 | ,000 | ,081 |

| Modèle | R | R-deux | R-deux ajusté | Erreur standard de l'estimation |
|---|---|---|---|---|
| | ,838 | ,702 | ,676 | ,11340 |

**50. Pour SNIAFFSA 4 :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | 21,259 | 11,052 | | ,065 | -1,418 | 43,937 |
| TP | -,249 | ,089 | -,386 | ,010 | -,432 | -,065 |
| AGE | ,132 | ,076 | ,214 | ,092 | -,023 | ,288 |
| GGLOB | ,987 | ,308 | ,455 | ,003 | ,355 | 1,618 |
| POIDS | -,073 | ,042 | -,219 | ,091 | -,159 | ,012 |

| Modèle | R | R-deux | R-deux ajusté | Erreur standard de l'estimation |
|---|---|---|---|---|
| | ,780 | ,608 | ,550 | 5,36161 |

**51. Pour SNIAFFSA 6 :**

| | Coefficients non standardisés | | Coefficients standardisés | Signification | Intervalle de confiance à 95% de B | |
|---|---|---|---|---|---|---|
| | B | Erreur standard | Bêta | | Borne inférieure | Borne supérieure |
| (constante) | ,501 | 3,705 | | ,893 | -7,115 | 8,118 |
| POIDS | -,223 | ,074 | -,685 | ,006 | -,376 | -,071 |
| BMI | ,551 | ,240 | ,520 | ,030 | ,059 | 1,044 |
| HYAPRI | -,150 | ,044 | -1,421 | ,002 | -,240 | -,061 |
| GLOPRI | 1,722 | ,249 | ,965 | ,000 | 1,211 | 2,234 |
| HYATRI | ,094 | ,027 | 1,313 | ,002 | ,039 | ,150 |

| Modèle | R | R-deux | R-deux ajusté | Erreur standard de l'estimation |
|---|---|---|---|---|
| | ,853 | ,727 | ,675 | 4,53214 |

**52. Pour SNIAFFSA 60 :**

| | Coefficients non standardisés | | Coefficients standardisés | Significafion | Intervalle de confiance à 95% de B | |
|---|---|---|---|---|---|---|
| | B | Erreur standard | Bêta | | Borne inférieure | Borne supérieure |
| (constante) | 1,774 | 2,805 | | ,533 | -4,016 | 7,564 |
| POIDS | -,204 | ,057 | -,725 | ,002 | -,322 | -,086 |
| BMI | ,489 | ,184 | ,537 | ,014 | ,109 | ,869 |
| HYAPRI | -,086 | ,036 | -,943 | ,026 | -,162 | -,011 |
| GLOPRI | 1,578 | ,193 | 1,029 | ,000 | 1,180 | 1,976 |
| HYATRI | ,049 | ,023 | , 784 | ,041 | ,002 | ,097 |

| Modèle | R | R-deux | R-deux ajusté | Erreur standard de l'estimation |
|---|---|---|---|---|
| | ,898 | ,806 | ,766 | 3,40834 |

D'autres avantages et caractéristiques de l'invention apparaîtront des exemples qui suivent, donnés à titre illustratif, et dans lesquels il sera fait référence aux dessins en annexe, dans lesquels :

- la figure 1 montre la courbe ROC obtenue à partir du score SNIFF 7bo pour la fibrose cliniquement significative. La C statistique (ou aire sous courbe ROC) est $0,910 \pm 0,016$.
- la figure 2 est une représentation des Box plots (médiane, quartiles et extrêmes) du score SNIFF 7bo à 7 variables versus le score F de Métavir (la référence est mesurée par la PBH),
- la figure 3 présente la distribution du score SNIFF 7bo à 7 variables versus le score F de Métavir (la référence est mesurée par la PBH),
- la figure 4 présente la distribution des groupes prédits ($\geq$ F2 : 0 : non, 1 : oui) du score SNIFF 7bo à 7 variables en fonction du score Metavir F,
- la figure 5 présente la performance diagnostique du score SNIFF 5 en fonction de sa valeur,
- la figure 6 présente la corrélation entre SNIAFF 5o à 5 variables et l'aire de fibrose. Ceci est à comparer à la Figure 3 (corrélation entre SNIFF 7bo à 7 variables et le score F) car ce sont les meilleurs indices pour les hépatopathies virales.
- la figure 7 présente la corrélation entre SNIFFA 4bo à 4 variables et le score F (Figure 7A) et entre SNIAFFA 4o à 4 variables et l'aire de fibrose (Figure 7B) (meilleurs indices pour les hépatopathies alcooliques), FCS : fibrose cliniquement significative.
- la figure 8 présente une comparaison des courbes ROC de Fibrotest 7 variables (C index : 0,839) et de SNIFF 7o à 7 variables (C index : 0,900) dans une même population de 238 malades. La différence est statistiquement significative ($p = 0,0036$ par la méthode de Hanley-McNeil).
- la figure 9 présente une comparaison des Box plots de Fibrotest à 7 variables et de SNIFF 7bo à 7 variables dans une même population de 238 malades avec hépatite virale. Les box plots de SNIFF 7bo sont plus bas pour les stades Métavir F0 et F1 et plus élevés pour les stades Métavir F2, F3 et F4 que ceux du Fibrotest 7 expliquant ainsi le meilleur pouvoir discriminant de SNIFF 7bo pour la fibrose cliniquement significative qui se détermine par rapport à la césure 0,50 pour Fibrotest et 0,29 pour SNIFF 7bo.

Exemple 1 Détermination d'un score SNIFF.

A. Patients.

Le patient avec une maladie chronique du foie a un prélèvement sanguin. Les variables biologiques sanguines simples sont déterminées selon les bonnes pratiques de laboratoire. Les résultats sont exprimés avec les unités précisées auparavant.

B. Méthodes de dosage.

La concentration en hyaluronate dans un échantillon sanguin est mesurée par une technique radioimmunoassay (Kabi-Pharmacia RIA Diagnostics, Uppsala, Sweden).

La concentration en $A_2M$ est déterminée par immunonéphelométrie laser utilisant un néphelomètre analyseur Behring. Le réactif est un antisérum de lapin anti-A2M humain.

Le taux de prothrombine est mesuré à partir du temps de Quick (TQ) qui est déterminé en ajoutant au plasma de la thromboplastine calcique (par exemple Neoplastin CI plus, Diagnostica Stago, Asnières, France) et le temps de coagulation est mesuré en secondes. Pour obtenir le taux de prothrombine (TP), une droite d'étalonnage est faite à partir de différentes dilutions d'un pool de plasma normaux estimé à 100%.

C. Calcul du score SNIFF.

Les résultats des variables isolées (ou simples) sont utilisés tels quels ou après transformation en variables combinatoires le cas échéant. L'ensemble de ces variables est inclus dans la formule de régression logistique. A titre d'exemple et sur la base des tableaux déjà décrit et d'un exemple d'utilisation de formule déjà décrit, l'homme du métier qui souhaite mettre en oeuvre le score SNIFF 4a à 4 marqueurs utilisera la formule suivante :

$p = 1/(1+\exp(-a_0 - a_1 (AH \text{ en } \mu g/l) - a_2 (TP \text{ en } \%) - a_3 (A2M \text{ en } mg/dl) - a_4 (AGE \text{ en } ans))$

Soit

$p = 1/(1+\exp(-2,365 - (0,011 * (AH \text{ en } \mu g/1)) - (-0,062 * (TP \text{ en } \%)) - (0,006 * (A2M \text{ en } mg/dl)) - (0,030 * (AGE \text{ en } ans)))$ Donnons deux exemples opposés :

| Cas | AH ($\mu$g/l) | TP (%) | A2M(mg/dl) | Age (ans) | Probabilité |
|-----|------|--------|------------|-----------|-------------|
| 1 | 273 | 90 | 374 | 64,0 | 0,981 |
| 5 | 25 | 89 | 157 | 30,2 | 0,273 |

Le cas 1 sera classé ayant une fibrose hépatique cliniquement significative et le cas 5 n'en ayant pas selon la césure fixée à 0,50.

Exemple 2 : Performances des scores de l'invention et comparaison des résultats obtenus avec les scores de l'invention et les méthodes de l'état de la technique.

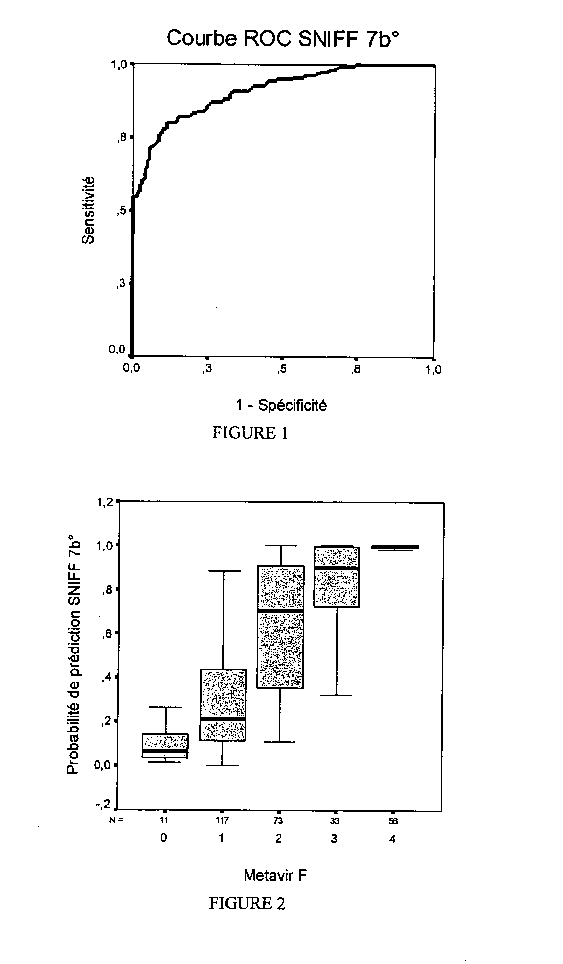

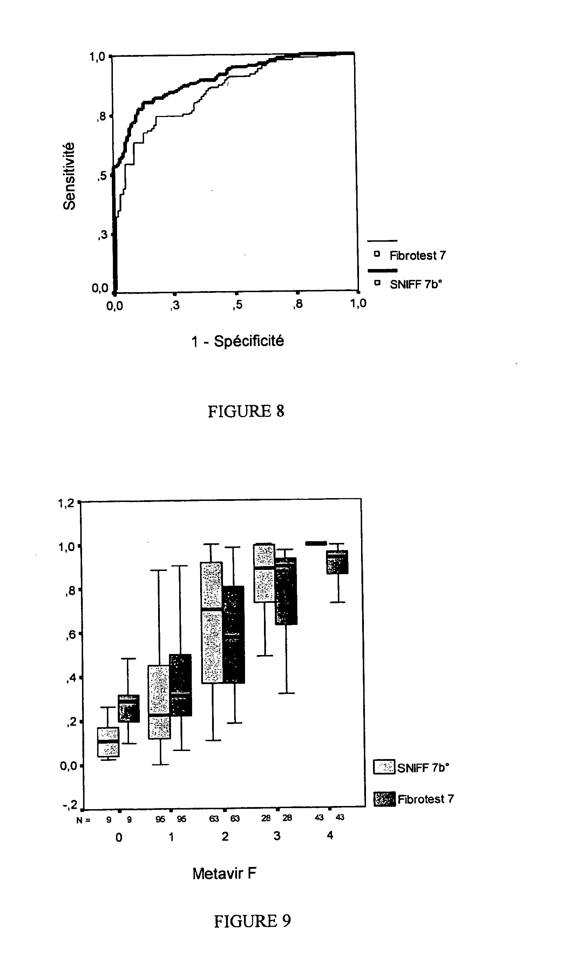

La courbe ROC (figure 1) représente la spécificité et la sensibilité en fonction de la valeur du test. Elle est mesurée par l'index C qui est considéré comme cliniquement pertinent à partir de 0,7. Plus la courbe se rapproche du coin supérieur gauche du cadre (spécificité et sensibilité de 100%) meilleure elle est. Ceci se mesure par l'aire sous courbe ROC (AUROC) appelée aussi C statistique. Il est possible de comparer ces AUROC d'où un intérêt supplémentaire qui permet de démontrer l'effet surprenant des scores SNIFF selon l'invention (figure 8).

L'index C obtenu dans le cadre des tests de l'invention a une valeur de $0,841 \pm 0,025$ pour le score SNIFF 5 et de $0,910 \pm 0,016$ pour le score SNIFF 7bo (figure 8). Ces index C sont donc cliniquement pertinents.

Les box plots présentés à la figure 2 montrent la distribution statistique des classes de SNIFF selon les stades Métavir F : médianes (trait noir horizontal gras), quartiles (limites haute et basse du rectangle gris) et extrêmes (barres horizontales aux extrémités). Il s'agit du score SNIFF 7bO.

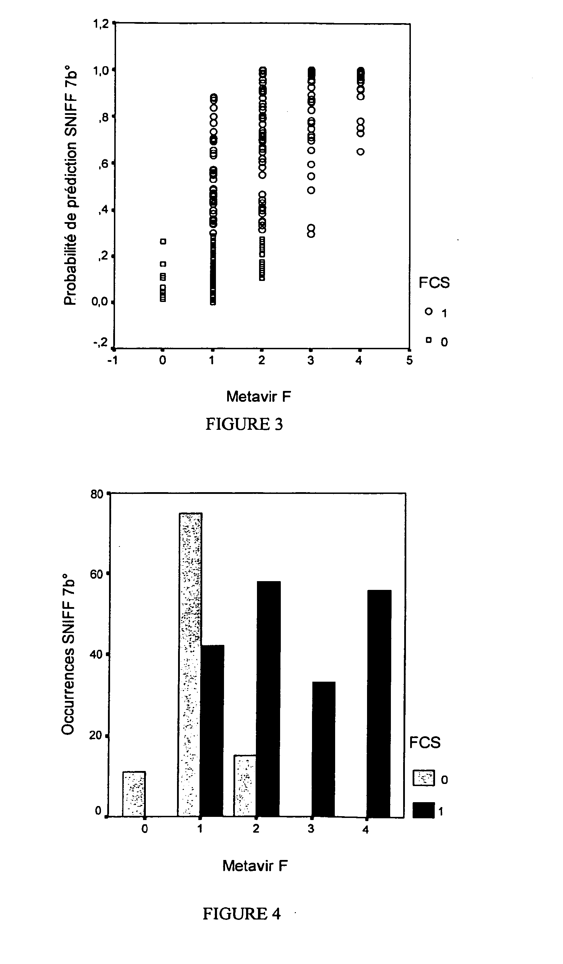

Dans la figure 3, il s'agit d'une même expression des résultats que dans la figure 2 mais elle montre les données brutes individuelles de SNIFF 7bo obtenu en mettant en oeuvre 7 variables en fonction du score Metavir F. Figurent aussi les groupes prédits : $\geq$ F2 : 0 (carré) : non, 1 : oui (cercle) (Figure 3). Cette figure permet de bien voir les chevauchements de score en particulier entre les stades F2 et F3 de Métavir. Par contre, dans les populations nombreuses, elle rend mal compte de la distribution du fait notamment des superpositions des valeurs individuelles.

La figure 4 est une expression différente de la figure précédente (Figure 3) où les malades sont regroupés par groupe prédit de fibrose cliniquement significative prédits : $\geq$ F2 : 0 (gris) : non, 1 : oui (noir). Ceci correspondait aux carrés et cercles, respectivement, de la figure 3. SNIFF ne classe mal aucun malade pour F0 et F4 et très peu pour F3 (aucun dans le cas de SNIFF 7bo de la figure 4). Autrement dit, en pratique SNIFF 7bo classe bien 100% des malades pour l'absence de fibrose ou la présence de cirrhose.

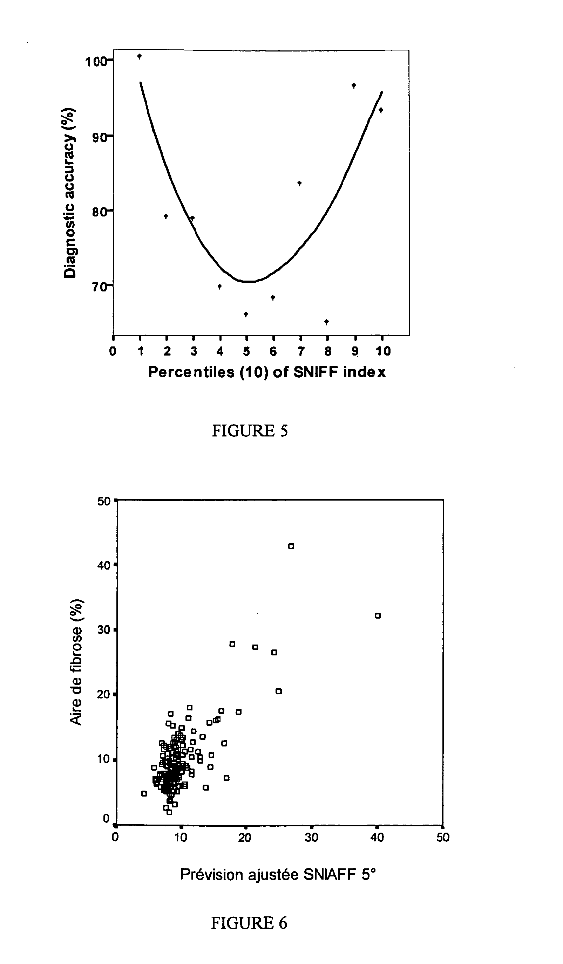

Comme on pouvait le deviner sur les figures précédentes, la figure 5 permet de bien voir que la performance diagnostique est excellente pour les valeurs basses et élevées et diminue pour les valeurs moyennes du score. Ainsi, la performance diagnostique est de 90,8% pour 50,0% des malades avec un score SNIFF 5 (figure 5).

Le score SNIFF 7 à 7 variables donne une estimation moins élevée de la fibrose : r = 0.769, p < $10^{-4}$ que l'index

SNIAFF 5o à 5 variables : r = 0.803, p < 10^{-4}.

Cette comparaison montre que l'estimateur SNIAFF de l'aire de fibrose (figure 6) est un indice plus fiable (précis) que le score SNIFF de fibrose.

De même le score SNIFFA 4bo à 4 variables donne une estimation moins élevée de la fibrose : r = 0.847, p < 10^{-4} que l'index SNIAFFA 4o à 4 variables : r = 0.914, p < 10^{-4}.

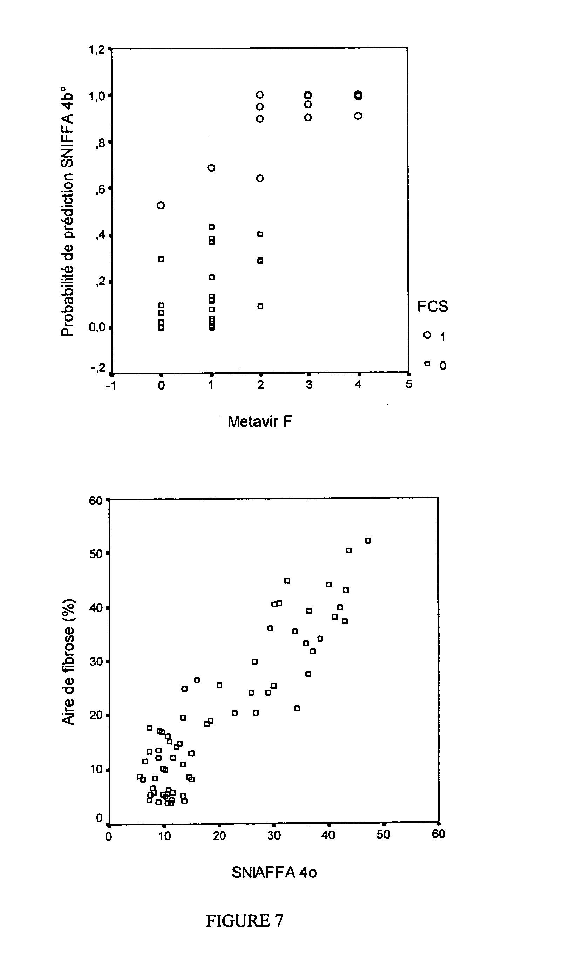

Cette comparaison montre aussi que l'estimateur SNIAFFA de l'aire de fibrose est un indice plus fiable (précis) que le score SNIFFA de fibrose (figure 7) également dans les hépatopathies alcooliques.

La comparaison des performances de SNIFF et Fibrotest montre que la performance diagnostique du Fibrotest 7 est de 74,2 % vs 82,1 % pour SNIFF 7. Les AUROC permettent de montrer que la différence de performance est statistiquement très significative (figure 8). La figure 9 montre de façon graphique le meilleur pouvoir discriminant de SNIFF 7 Sur Fibrotest 7.

REFERENCES

1. Oberti F, Valsesia E, Pilette C, Rousselet M, Bedossa P, Aubé C et al, Calès P. Noninvasive diagnosis of hepatic fibrosis or cirrhosis. Gastroenterology 1997;113:1609-16.
2. Croquet V, Vuillemin E, Ternisien C, Pilette C, Oberti F, Gallois Y, Trossaert M, Rousselet MC, Chappard D, Calès P. Prothrombin index is an indirect marker of severe liver fibrosis. Eur J Gastroenterol Hepatol 2002;14: 1133-41.
3. Pilette C, Calès P. Existe-t-il des marqueurs sanguins de fibrose hépatique utilisables en pratique clinique ? Rev Med Interne 2002;23:885-8.
4. Pilette C, Rousselet M, Bedossa P, Chappard D, Oberti F, Rifflet H et al, Calès P. Histopathological evaluation of liver fibrosis: quantitative image analysis vs semi-quantitative scores : comparison with serum markers. J Hepatol 1998; 28:439-46.
5. Aubé C, Oberti F, Korali N, Korali N, Namour A, L et al, Calès P. Ultrasonographic diagnosis of hepatic fibrosis or cirrhosis. J Hepatol 1999; 30:472-8.
6. Moal F, Chappard D, Wang J, Vuillemin E, Michalak-Provost S, Rousselet MC, Oberti F, Calès P. Fractal dimension can distinguish models and pharmacological changes in liver fibrosis in rats. Hepatology 2002;36:840-9.
7. Michalak S, Rousselet MC, Bedossa P, Pilette C, Chappard D, Oberti F, Gallois Y, Calès P. Respective role of porto-septal fibrosis and centrolobular fibrosis in alcoholic liver diseases. J Pathol 2003;201:55-62.

**Revendications**

**1.** Méthode pour diagnostiquer la présence et/ou la sévérité d'une pathologie hépatique et/ou pour suivre l'efficacité d'un traitement curatif contre une pathologie hépatique chez un sujet comprenant l'établissement d'au moins un score diagnostique non-invasif, en particulier d'un score diagnostique de la fibrose portale et septale et/ou d'un estimateur de l'aire de fibrose, et/ou d'un estimateur de la dimension fractale, par la mise en oeuvre des étapes suivantes :

a') pour la détermination de l'aire de fibrose ou de la dimension fractale, mesurer dans un échantillon dudit sujet au moins une variable choisie dans le groupe constitué par alpha-2 macroglobuline (A2M), acide hyaluronique (AH ou hyaluronate), propeptide N-terminal du procollagène de type III (P3P), gamma-glutamyltranspeptidase (GGT), bilirubine, gamma-globulines (GLB), plaquettes (PLQ), taux de prothrombine (TP), aspartate amino-transférase (ASAT), alanine amino-transférase (ALAT), urée, sodium (NA), glycémie, triglycérides, albumine (ALB), phosphatases alcalines (PAL), YKL-40 (human cartilage glycoprotein 39), Matrix metalloproteinase 2 (MMP-2), Ferritine,

a) pour l'établissement d'un score diagnostique de la fibrose portale et septale, mesurer dans un échantillon dudit sujet au moins trois variables choisies dans le groupe constitué par alpha-2 macroglobuline (A2M), acide hyaluronique (AH ou hyaluronate), apolipoprotéine A1 (ApoA1), propeptide N-terminal du procollagène de type III (P3P), gamma-glutamyltranspeptidase (GGT), bilirubine, gamma-globulines (GLB), plaquettes (PLQ), taux de prothrombine (TP), aspartate amino-transférase (ASAT), alanine aminotransférase (ALAT), urée, sodium (NA), glycémie, triglycérides, albumine (ALB), phosphatases alcalines (PAL), YKL-40 (human cartilage glycoprotein 39), Matrix metalloproteinase 2 (MMP-2), Ferritine,

b) éventuellement, recueillir au moins une variable clinique caractérisant ledit sujet ; pour le score diagnostique de la fibrose portale et septale, les étapes a) et b) ci-dessus étant telles que au moins 4 variables sont mesurées ou recueillies,

c) combiner, dans une fonction logistique ou linéaire, lesdites variables afin d'obtenir un score diagnostique de la fibrose portale et septale et/ou d'un estimateur diagnostique de l'aire de fibrose, et/ou d'un estimateur diagnostique de la dimension fractale ;

d) diagnostiquer la présence et/ou la sévérité de ladite pathologie et/ou l'efficacité dudit traitement à partir du score obtenu lors de la combinaison de l'étape (c).

**2.** Méthode selon la revendication 1, **caractérisée en ce que** les variables cliniques caractérisant le sujet sont choisies parmi le poids corporel (poids), l'index de masse corporelle, l'âge (âge) à la date du recueil de l'échantillon et la cause.

**3.** Méthode selon l'une quelconque des revendications 1 ou 2, **caractérisée en ce que** préalablement à l'étape (c), les variables mesurées à l'étape (a) ou (a') et les variables recueillies à l'étape (b) sont combinées les unes aux autres.

**4.** Méthode selon l'une quelconque des revendications 1 à 3, **caractérisée en ce que** ladite pathologie hépatique est choisie parmi les maladies hépatiques d'origine virale, les maladies hépatiques d'origine alcoolique et les stéatoses.

**5.** Méthode selon l'une quelconque des revendications 1 à 4, **caractérisée en ce que** dans l'étape (a) de ladite méthode, on mesure dans un échantillon dudit sujet 4, 5, 6 ou 7 variables.

**6.** Méthode selon l'une quelconque des revendications 1 à 5, **caractérisée en ce que** l'on combine, à l'étape (c) de ladite méthode, les variables a-2 macroglobuline (A2M) et taux de prothrombine (TP) et au moins deux variables choisies parmi plaquettes (PLQ), aspartate aminotransférase (ASAT), urée, acide hyaluronique (AH) et âge.

**7.** Méthode selon la revendication 6, **caractérisée en ce que** l'on combine à l'étape (c) :

- a-2 macroglobuline (A2M), taux de prothrombine (TP), acide hyaluronique (AH) et âge (score appelé SNIFF 4a) ;
- a-2 macroglobuline (A2M), taux de prothrombine (TP), aspartate amino-transférase (ASAT) et âge (score appelé SNIFF 4b) ;
- a-2 macroglobuline (A2M), taux de prothrombine (TP), plaquettes (PLQ), aspartate amino-transférase (ASAT) et âge (score appelé SNIFF 5) ;
- a-2 macroglobuline (A2M), taux de prothrombine (TP), plaquettes (PLQ), aspartate amino-transférase (ASAT), urée et acide hyaluronique (AH) (score appelé SNIFF 6) ; ou
- a-2 macroglobuline (A2M), taux de prothrombine (TP), plaquettes (PLQ), aspartate amino-transférase (ASAT), urée, acide hyaluronique (AH) et âge (score appelé SNIFF 7).

**8.** Méthode selon l'une quelconque des revendications **1 à 5, caractérisée en ce que** l'on combine, à l'étape (c) de ladite méthode, outre la variable taux de prothrombine (TP), au moins trois variables choisies parmi aspartate amino-transférase (ASAT), alanine amino-transférase (ALAT) et phosphatases alcalines (PAL), âge, acide hyaluronique (AH ou hyaluronate) et a-2 macroglobuline (A2M).

**9.** Méthode selon la revendication 8, **caractérisée en ce que** l'on combine 5 à l'étape (c) :

- taux de prothrombine (TP), aspartate aminotransférase (ASAT), alanine amino-transférase (ALAT) et phosphatases alcalines (PAL) (score appelé SNIFFA 4b),ou
- taux de prothrombine (TP), âge, acide hyaluronique (AH ou hyaluronate) et a-2 macroglobuline (A2M) (score appelé SNIFFA 4c).

**10.** Méthode selon l'une quelconque des revendications **1 à 5, caractérisée en ce que** l'on combine, à l'étape (c) de ladite méthode, au moins les 4 variables suivantes :

acide hyaluronique (AH ou hyaluronate), gammaglutamyltranspeptidase (GGT), bilirubine et plaquettes (PLQ).

**11.** Méthode selon la revendication **10, caractérisée en ce que** l'on combine, à l'étape (c), en plus au moins deux variables et de manière encore préférée, au moins quatre variables choisies parmi a-2 macroglobuline (A2M), urée, et gamma-globulines (GLB).

**12.** Méthode selon l'une quelconque des revendications **1 à 5, caractérisée en ce que** l'on combine au moins les quatre variables suivantes : plaquettes (PLQ), taux de prothrombine (TP), aspartate amino-transférase (ASAT) et âge.

**13.** Méthode selon l'une quelconque des revendications **1** à **5, caractérisée en ce que** l'on combine à l'étape (c) de ladite méthode, outre la variable taux de prothrombine (TP), au moins trois variables choisies parmi aspartate amino-transférase (ASAT), triglycérides, âge et glycémie.

**14.** Méthode selon l'une quelconque des revendications **1** à **5, caractérisée en ce que** l'on combine à l'étape (c) de ladite méthode au moins cinq des 6 variables suivantes : a-2 macroglobuline (A2M), plaquettes (PLQ), taux de prothrombine (TP), urée, acide hyaluronique (AH ou hyaluronate) ou cause.

**15.** Méthode selon l'une quelconque des revendications **1** à **5, caractérisée en ce que** l'on combine à l'étape (c) de ladite méthode de la présente invention, outre la variable taux de prothrombine (TP), au moins trois, de préférence au moins quatre ou, de façon encore préférée, cinq ou six variables choisies parmi plaquettes (PLQ), urée, acide hyaluronique (AH ou hyaluronate), bilirubine, a-2 macroglobuline (A2M), gamma-glutamyltranspeptidase (GGT), gamma-globulines (GLB), aspartate amino-transférase (ASAT) et cause.

Courbe ROC SNIFF 7b°

FIGURE 1

Metavir F

FIGURE 2

FIGURE 3

FIGURE 4

FIGURE 5

FIGURE 6

FIGURE 7

FIGURE 8

Metavir F

FIGURE 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0216949 A

- WO 03073822 A

**Littérature non-brevet citée dans la description**

- **BEDOSSA et al.** *Hepatology,* 1994, vol. 20, 15-20
- **PILETTE et al.** *J Hepatol,* 1998, vol. 28, 439-46
- **IMBERT-BISMUT et al.** *Lancet,* 2001, vol. 37, 1069-1075
- **MOAL F et al.** *Hepatology,* 2002, vol. 36, 840-9
- **WAI et al.** *Hepatology,* 2003, vol. 38, 518-526
- **OBERTI F ; VALSESIA E ; PILETTE C ; ROUSSELET M ; BEDOSSA P ; AUBÉ C ; CALÈS P. et al.** Noninvasive diagnosis of hepatic fibrosis or cirrhosis. *Gastroenterology,* 1997, vol. 113, 1609-16
- **CROQUET V ; VUILLEMIN E ; TERNISIEN C ; PILETTE C ; OBERTI F ; GALLOIS Y ; TROSSAERT M ; ROUSSELET MC ; CHAPPARD D ; CALÈS P.** Prothrombin index is an indirect marker of severe liver fibrosis. *Eur J Gastroenterol Hepatol,* 2002, vol. 14, 1133-41
- **PILETTE C ; CALÈS P.** Existe-t-il des marqueurs sanguins de fibrose hépatique utilisables en pratique clinique ?. *Rev Med Interne,* 2002, vol. 23, 885-8

- **PILETTE C ; ROUSSELET M ; BEDOSSA P ; CHAPPARD D ; OBERTI F ; RIFFLET H ; CALÈS P. et al.** Histopathological evaluation of liver fibrosis: quantitative image analysis vs semi-quantitative scores : comparison with serum markers. *J Hepatol,* 1998, vol. 28, 439-46
- **AUBÉ C ; OBERTI F ; KORALI N ; KORALI N ; NAMOUR A, L ; CALÈS P et al.** Ultrasonographic diagnosis of hepatic fibrosis or cirrhosis. *J Hepatol,* 1999, vol. 30, 472-8
- **MOAL F ; CHAPPARD D ; WANG J ; VUILLEMIN E ; MICHALAK-PROVOST S ; ROUSSELET MC ; OBERTI F ; CALÈS P.** Fractal dimension can distinguish models and pharmacological changes in liver fibrosis in rats. *Hepatology,* 2002, vol. 36, 840-9
- **MICHALAK S ; ROUSSELET MC ; BEDOSSA P ; PILETTE C ; CHAPPARD D ; OBERTI F ; GALLOIS Y ; CALÈS P.** Respective role of porto-septal fibrosis and centrolobular fibrosis in alcoholic liver diseases. *J Pathol,* 2003, vol. 201, 55-62